(19) Europäisches Patentamt
European Patent Office
Office européen des brevets

(11) EP 0 680 950 B1

(12) **EUROPEAN PATENT SPECIFICATION**

(45) Date of publication and mention of the grant of the patent:
**23.05.2001 Bulletin 2001/21**

(21) Application number: **95900905.1**

(22) Date of filing: **16.11.1994**

(51) Int Cl.[7]: **C07D 209/12**, C07D 209/30, C07D 401/06, A61K 31/40, A61K 31/44, C07D 405/06

(86) International application number:
**PCT/JP94/01934**

(87) International publication number:
**WO 95/14003 (26.05.1995 Gazette 1995/22)**

# (54) PROPENONE DERIVATIVES

PROPENON-DERIVATE

DERIVES DE PROPENONE

(84) Designated Contracting States:
**AT BE CH DE DK ES FR GB GR IE IT LI LU MC NL PT SE**

(30) Priority: **17.11.1993 JP 28809193**

(43) Date of publication of application:
**08.11.1995 Bulletin 1995/45**

(73) Proprietor: **KYOWA HAKKO KOGYO CO., LTD.**
**Chiyoda-ku, Tokyo 100 (JP)**

(72) Inventors:
- **IKEDA, Shun-ichi**
  **Shizuoka 410 (JP)**
- **KIMURA, Uichiro**
  **Sunto-gun, Shizuoka 411 (JP)**
- **ASHIZAWA, Tadashi**
  **Shizuoka 410 (JP)**
- **GOMI, Katsushige**
  **Shizuoka 410-11 (JP)**
- **SAITO, Hiromitsu**
  **Kawasaki-shi, Kanagawa 214 (JP)**
- **KASAI, Masaji**
  **Kanagawa 251 (JP)**

(74) Representative: **VOSSIUS & PARTNER**
**Siebertstrasse 4**
**81675 München (DE)**

(56) References cited:
**FR-A- 2 230 349**
**JP-T- 5 507 072**
**US-A- 5 196 446**

- **Farmaco, Ed. Sci., 26(7), 591-6 (1971).**
- **Zh. Prik. Spektrosk., 31(6), 1036-41 (1979).**
- **Khim. Geterotsikl. Soedin., 1974(4), 519-24.**
- **Khim.-Farm. Zh., 23(2), 201-6 (1989).**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 680 950 B1

Printed by Jouve, 75001 PARIS (FR)

## Description

Technical Field

The present invention relates to propenone derivatives having an antitumor activity.

Background Art

Typical examples of compounds having an antitumor activity include mitomycin C, adriamycin, vincristine and the like, all of which are clinically used as useful anticancer agents. However, since each of the compounds also has adverse effects such as myelotoxicity, cardiotoxicity, nerve damage, etc., a novel anticancer agent having less adverse effects is demanded.

Chalcone derivatives are known as having the activity to inhibit polymerization of tubulin and an anticancer activity [Journal of the Medicinal Chemistry (J. Med. Chem.), 33, 1948 (1990) and U. S. Patent No. 4904697]. Compounds represented by the formula (I) below in which $R^1$ is phenyl, $R^2$ is methyl, $R^3$ is hydrogen, $R^4$ is hydrogen, $R^5$ is methoxy, $R^6$ is hydrogen and $R^7$ is hydrogen are known as 3-(indol-3-yl)-1-(3,4,5-trimethoxyphenyl)-2-propen-1-one derivatives (French Patent No. 2230349). 3-(Indol-3-yl)-1-phenyl-2-propen-1-one derivatives in which the 3,4,5-trimethoxyphenyl group is substituted by a benzene ring having other substituents or an unsubstituted benzene ring are disclosed in the above French Patent, Kim. Geterotsikl. Soedin, 1066 (1970) and Kim. Geterotsikl. Soedin, 268 (1969).

In WO91/16305 indolyl derivatives are disclosed as PTK inhibitors.

Disclosure of the Invention

The present invention relates to propenone derivatives represented by the following formula (I):

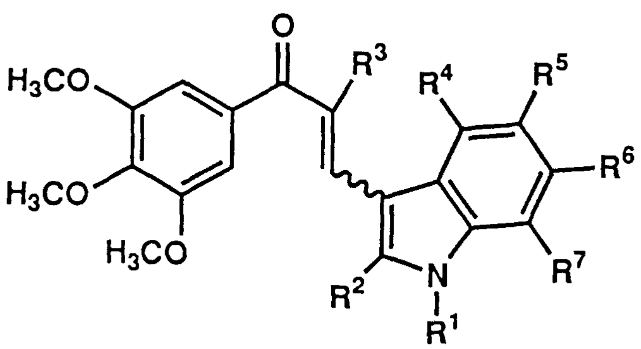

(I)

wherein $R^1$ represents hydrogen, lower alkyl, lower alkanoyl, lower alkoxycarbonyl, lower alkylsulfonyl, aralkyl, substituted or unsubstituted aroyl, substituted or unsubstituted arylsulfonyl, substituted or unsubstituted heteroarylcarbonyl, substituted or unsubstituted heteroarylsulfonyl, diglycolyl or

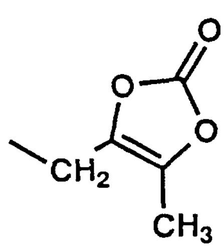

;

$R^2$ represents hydrogen, lower alkyl, halogen, substituted or unsubstituted aryl, or substituted or unsubstituted heteroaryl; $R^3$ represents hydrogen, lower alkyl or substituted or unsubstituted aryl; and $R^4$, $R^5$, $R^6$ and $R^7$ represent independently hydrogen, lower alkyl, lower alkoxy, aralkyloxy, hydroxy, nitro, halogen, trifluoromethyl or $-NR^8R^9$ (wherein $R^8$ and $R^9$ represent independently hydrogen, lower alkyl, lower alkanoyl, lower alkoxycarbonyl or substituted or unsubstituted aroyl), or pharmaceutically acceptable salts thereof.

Compounds represented by the formula (I) are hereinafter referred to as Compounds (I). Compounds (Ia), (I-1) and (Ia-1) are included in Compounds (I). The same applies to the compounds represented by other formula numbers.

In the definitions of the groups of Compounds (I), the lower alkyl means a straight-chain or branched alkyl group having 1 to 6 carbon atoms, such as methyl, ethyl, propyl, isopropyl, butyl, 2-butyl, isobutyl, tert-butyl, 1-pentyl, 2-pentyl, 3-pentyl, isoamyl and hexyl. The lower alkyl moiety of the lower alkoxycarbonyl, lower alkylsulfonyl and lower alkoxy has the same meaning as the lower alkyl defined above. The lower alkanoyl means a straight-chain or branched alkanoyl group having 1 to 7 carbon atoms, such as formyl, acetyl, propionyl, butyryl, isobutyryl, valeryl, isovaleryl, pivaloyl, hexanoyl and heptanoyl. The aralkyl means an aralkyl group having 7 to 13 carbon atoms, such as benzyl, phenethyl, benzhydryl and naphthylmethyl. The aralkyl moiety of the aralkyloxy has the same meaning as the aralkyl defined above. The aryl means phenyl and naphthyl, and the aryl moiety of the aroyl and arylsulfonyl has the same meaning as the aryl defined above. The heteroaryl means pyridyl, pyrazyl, pyrimidinyl, pyridazinyl, pyrrolyl, imidazolyl, pyrazolyl oxazolyl, thiazolyl, furyl, thienyl, quinolyl, benzimidazolyl, indolyl and the like. The heteroaryl moiety of the heteroarylcarbonyl and heteroarylsulfonyl has the same meaning as the heteroaryl defined above. The halogen includes fluorine, chorine, bromine and iodine.

The substituted aroyl, substituted arylsulfonyl, substituted heteroarylcarbonyl, substituted heteroarylsulfonyl, substituted aryl and substituted heteroaryl each have the same or different 1 to 3 substituents such as lower alkyl, lower alkoxy, lower alkanoyl, lower alkoxycarbonyl, hydroxy, amino, lower alkylamino, di(lower alkyl)amino, lower alkanoylamino, nitro, trifluoromethyl and halogen. In the definitions of the substituents, the lower alkyl moiety of the lower alkylamino and di(lower alkyl)amino has the same meaning as the lower alkyl defined above, the lower alkanoyl moiety of the lower alkanoylamino has the same meaning as the lower alkanoyl defined above, and the lower alkyl, lower alkoxy, lower alkanoyl, lower alkoxycarbonyl and halogen each has the same meaning as defined above.

The pharmaceutically acceptable salts of Compounds (I) include inorganic acid addition salts such as hydrochloride, sulfate and phosphate, and organic acid addition salts such as maleate, fumarate, succinate, tartrate, citrate, oxalate and methanesulfonate.

The present invention is described in detail below.

In the processes shown below, if the defined groups are converted under the conditions of the processes or are not suitable for carrying out the processes, the processes can be readily carried out by applying thereto means conventionally used in organic synthetic chemistry, for example, a means such as protection or deprotection of functional groups, or a method such as oxidation, reduction or hydrolysis.

Compound (I) can be prepared according to the following reaction step.

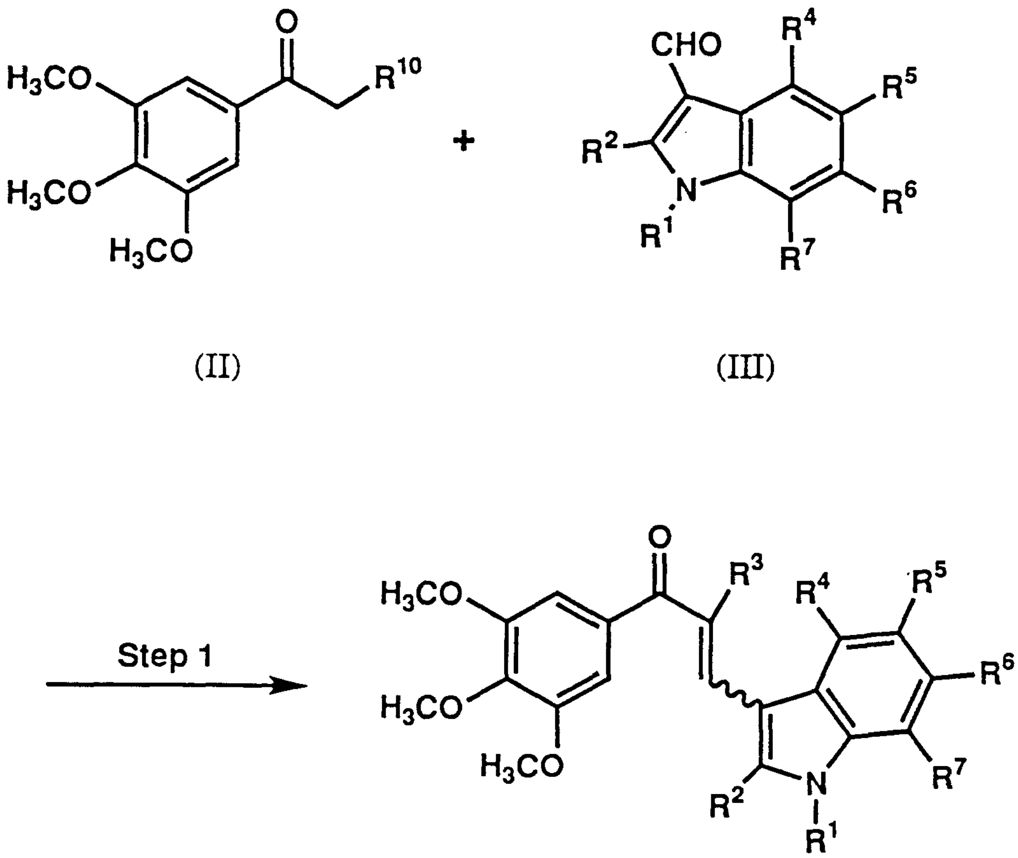


(II) (III)

(I)

(In the formulae, $R^{10}$ represents hydrogen, lower alkyl or

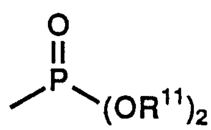

wherein $R^{11}$ represents lower alkyl; and $R^1$, $R^2$, $R^3$, $R^4$, $R^5$, $R^6$ and $R^7$ have the same meanings as defined above.)

In the definitions of $R^{10}$ and $R^{11}$, the lower alkyl has the same meaning as defined above.

Step 1

Compound (I) can be obtained by reacting Compound (II) with Compound (III) in the presence of a base in an inert solvent. As the base, inorganic bases such as potassium carbonate, sodium carbonate and cesium fluoride, quaternary ammonium fluorides such as tetra-n-butylammonium fluoride, secondary amines such as piperidine, pyrrolidine and morpholine, metal alkoxides such as potassium tert-butoxide, metal amides such as lithium diisopropylamide, metal hydrides such as sodium hydride and the like may be used in an amount of 0.01 to 10 equivalents. As the solvent, aprotic solvents (for example, ethyl acetate and tetrahydrofuran), aromatic hydrocarbons (for example, toluene), halogenated hydrocarbons (for example, chloroform), alcohols (for example, methanol and ethanol) and the like may be used alone or in combination. The reaction is carried out at - 78°C to the boiling point of the solvent employed in the reaction, and is completed in 0.1 hour to 7 days.

The starting Compound (II) is commercially available or can be prepared according to the following reaction steps.

Compound (IIa) which is the starting Compound (II) in which $R^{10}$ is lower alkyl can be prepared according to the following reaction steps.

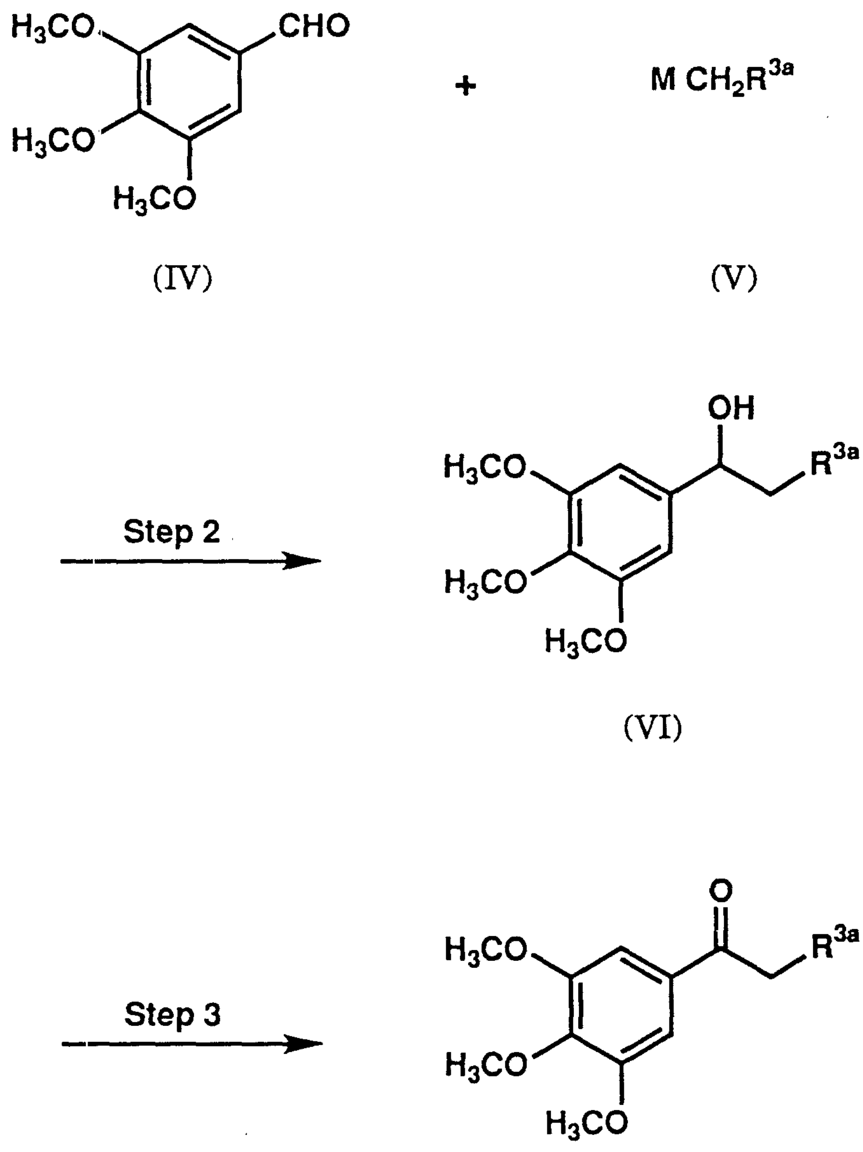


(IV) (V) (VI) (IIa)

(In the formulae, $R^{3a}$ represents lower alkyl; and M represents alkali metal, alkaline earth metal halide or cerium dichloride.)

In the definition of $R^{3a}$, the lower alkyl has the same meaning as defined above. In the definition of M, the alkali metal means lithium, sodium, potassium or cesium, and the alkaline earth metal halide means magnesium chloride, magnesium bromide or magnesium iodide.

Step 2

Compound (IV) can be obtained by reacting 3,4,5-trimethoxybenzaldehyde (IV) with 1 to 2 equivalents of Compound (V) in an inert solvent. As the solvent, aprotic solvents (for example, ethyl acetate and tetrahydrofuran), aromatic hydrocarbons (for example, toluene) and the like may be used alone or in combination. The reaction is carried out at - 100 to 30°C, and is completed in 0.1 to 5 hours.

Step 3

Compound (IIa) can be obtained by treating Compound (VI) in the presence of an oxidizing agent in an inert solvent. As the oxidizing agent, 1 to 50 equivalents of chromium trioxide, a pyridine complex or hydrochloric acid complex thereof, potassium dichromate, manganese dioxide, 2,3-dichloro-5,6-dicyanobenzoquinone and the like may be used. As the solvent, aprotic solvents (for example, acetone and N,N-dimethylformamide), halogenated hydrocarbons (for example, dichloromethane and chloroform), acetic acid, sulfuric acid, water and the like may be used alone or in combination. The reaction is carried out at - 10°C to the boiling point of the solvent employed in the reaction, and is completed in 0.1 to 48 hours.

Compound (IIc) which is the starting Compound (II) in which $R^{10}$ is

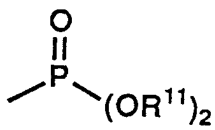

wherein $R^{11}$ has the same meaning as defined above can be prepared according to the following reaction steps.

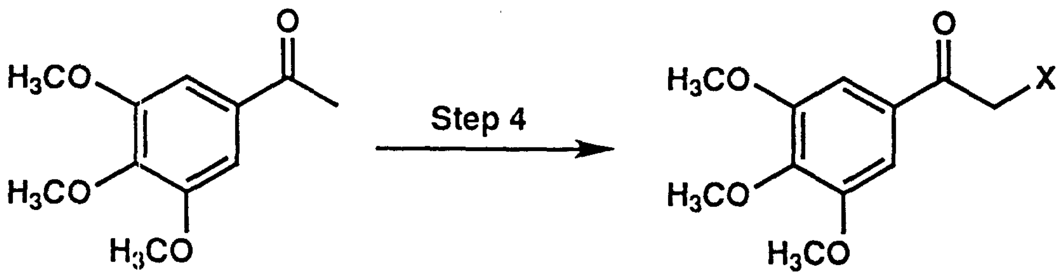


(IIb) (VII)

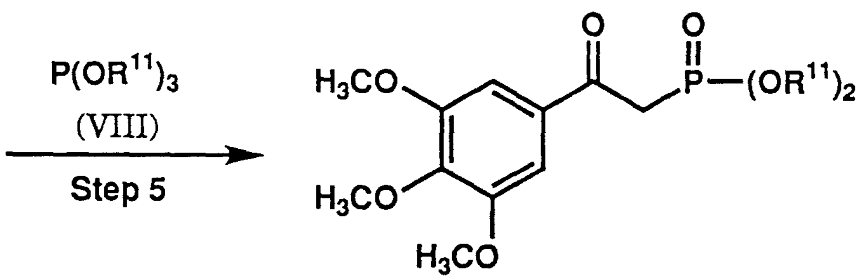


(IIc)

(In the formulae, X represents halogen, and $R^{11}$ has the same meaning as defined above.)

In the definition of X, the halogen has the same meaning as defined above.

Step 4

Compound (VII) can be obtained by treating Compound (IIb), which is Compound (II) in which $R^{10}$ is hydrogen, in the presence of a halogenating agent in an inert solvent. As the halogenating agent, 1 to 5 equivalents of pyrrolidone hydrotribromide, tetra-n-butylammonium tribromide, bromine and the like may be used. As the solvent, aprotic solvents (for example, ethyl acetate and tetrahydrofuran), acetic acid, water and the like may be used alone or in combination.

The reaction is carried out at 0°C to the boiling point of the solvent employed in the reaction, and is completed in 0.5 to 12 hours.

Step 5

Compound (IIc) can be obtained by reacting Compound (VII) with Compound (VIII) in an inert solvent or without a solvent. As the solvent, aprotic solvents (for example, ethyl acetate, tetrahydrofuran and N,N-dimethylformamide), aromatic hydrocarbons (for example, toluene), halogenated hydrocarbons (for example, chloroform) and the like may be used alone or in combination. The reaction is carried out at 50 to 250°C, and is completed in 0.1 hour to 7 days.

The starting Compound (III) is commercially available or can be prepared according to the method described or cited in The Chemistry of Heterocyclic Compounds, Indoles, 3 (9) (1972, John Wiley and Sons Incorporated) or the following reaction step.

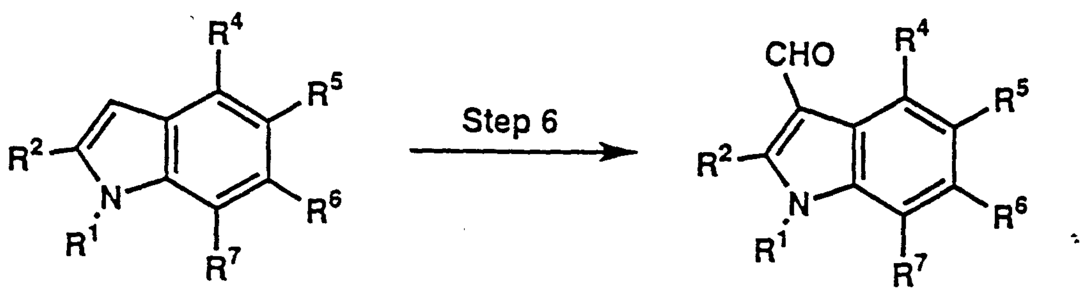


(IX) (III)

(In the formulae, $R^1$, $R^2$, $R^4$, $R^5$, $R^6$ and $R^7$ have the same meanings as defined above.)

Step 6

Compound (III) can be obtained by reacting Compound (IX) in the presence of a formylating agent in an inert solvent, and then, if necessary, treating the reaction product with a base. As the formylating agent, 1 to 10 equivalents of Vilsmeyer reagent prepared from N,N-dimethylformamide, phosphorus oxychloride and the like may be used. As the solvent, aprotic solvents (for example, tetrahydrofuran and N,N-dimethylformamide), aromatic hydrocarbons (for example, toluene), halogenated hydrocarbons (for example, chloroform) and the like may be used alone or in combination. As the base, 5 to 100 equivalents of an aqueous solution of sodium hydroxide and the like may be used. The reaction is carried out at 0°C to the boiling point of the solvent employed in the reaction, and is completed in 0.01 to 6 hours.

The starting Compound (IX) in Step 6 is commercially available or can be prepared according to the method described or cited in The Chemistry of Heterocyclic Compounds, Indoles, 2 and 3 (1972, John Wiley and Sons Incorporated). 6-Trifluoromethylindole and 6-isopropylindole can be prepared according to the method described in Israel Journal of Chemistry (Israel J. Chem.), 4, 155 (1966) and Organic Synthesis (Org. Syn.), 63, 214 (1985), respectively. Compound (IXa) which is the starting Compound (IX) in which $R^1$, $R^2$, $R^4$, $R^5$ and $R^7$ are each hydrogen, and $R^6$ is $R^{6a}CH_2$ (In the formula, $R^{6a}$ represents lower alkyl having 2 to 6 carbon atoms. In the definition of $R^{6a}$, the lower alkyl having 2 to 6 carbon atoms has the same meaning as the lower alkyl having 2 to 6 carbon atoms within the definition of the lower alkyl defined above.) can be prepared according to the following reaction steps.

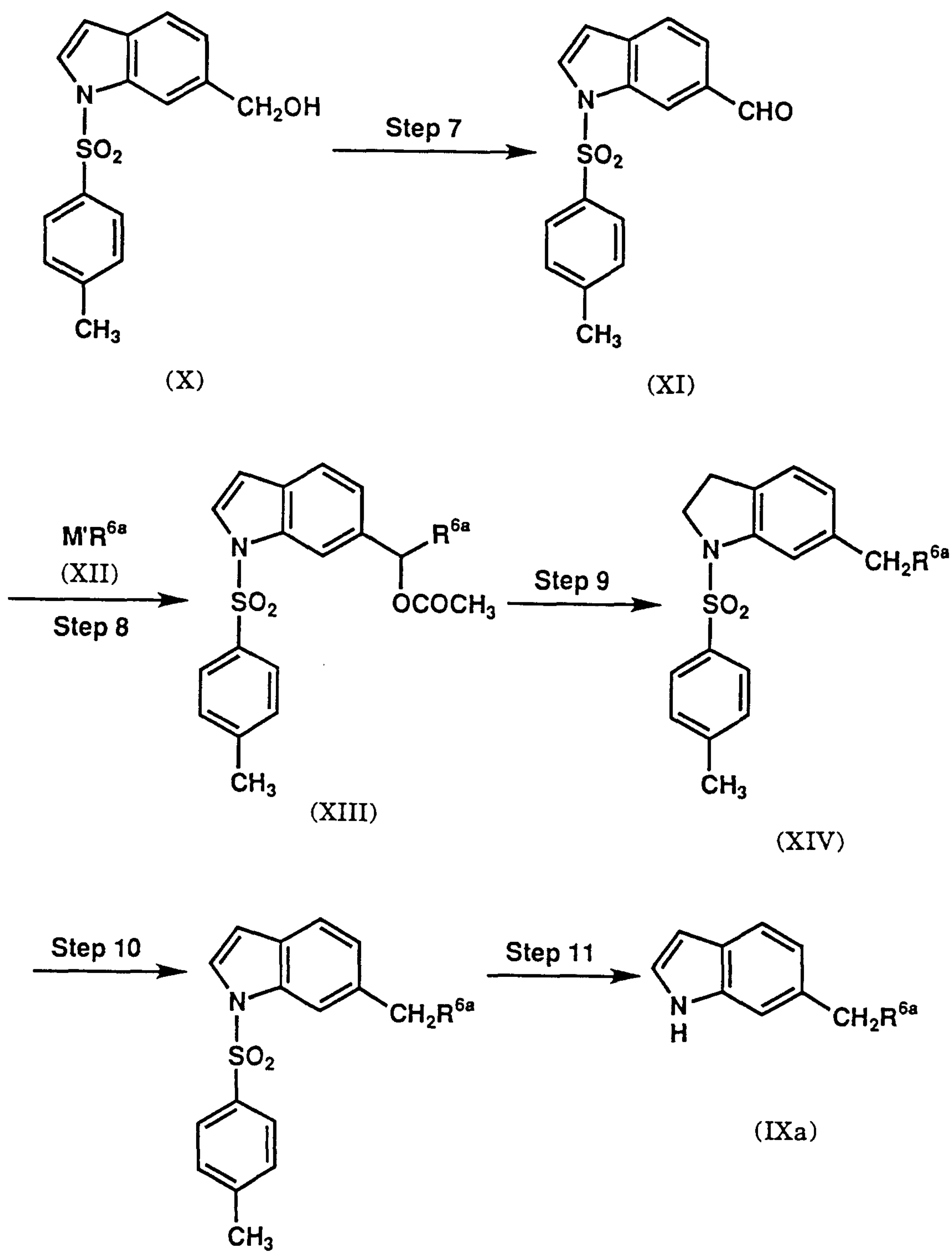


(X) (XI) (XIII) (XIV) (XV) (IXa)

(In the formulae, M' represents alkali metal, alkaline earth metal halide or cerium dichloride, and $R^{6a}$ represents the same as defined above.)

In the definition of M', the alkali metal and alkali earth metal halide have the same meanings as defined above.

Step 7

Compound (XI) can be obtained by oxidizing, in the same manner as Process 3, 6-hydroxymethyl-1-toluenesulfonylindole [Compound (X)] obtained according to the method described in U. S. Patent No. 4894386.

Step 8

Compound (XIII) can be obtained by reacting Compound (XI) with Compound (XII) in an inert solvent, and then treating the reaction product with acetic anhydride. As the solvent, aprotic solvents (for example, ethyl acetate and tetrahydrofuran), and aromatic hydrocarbons (for example, toluene) are used alone or in combination. The reaction is carried out at - 100 to 30°C, and is completed in 0.1 to 5 hours.

Step 9

Compound (XIV) can be obtained by treating Compound (XIII) in the presence of a catalyst in an inert solvent under an atmosphere of hydrogen. As the catalyst, palladium, platinum and the like may be used alone or held on activated carbon, barium sulfate, aluminum oxide or the like in an amount of 1 to 500 weight % of the amount of Compound (XIII). As the solvent, aprotic solvents (for example, ethyl acetate and tetrahydrofuran), aromatic hydrocarbons (for example, toluene), protic solvents (for example, methanol, ethanol and acetic acid) and the like may be used alone or in combination. The reaction is carried out at 10 to 120°C, and is completed in 1 to 50 hours.

Step 10

Compound (XV) can be obtained by treating Compound (XIV) in the presence of an oxidizing agent in an inert solvent. As the oxidizing agent, 1 to 500 equivalents of manganese dioxide, 2,3-dichloro-5,6-dicyanobenzoquinone and the like may be used. As the solvent, aprotic solvents (for example, ethyl acetate and tetrahydrofuran), aromatic hydrocarbons (for example, toluene and chlorobenzene), protic solvents (for example, methanol, ethanol and acetic acid) and the like may be used alone or in combination. The reaction is carried out at 50 to 150°C, and is completed in 2 to 250 hours.

Step 11

Compound (IXa) can be obtained by treating Compound (XV) in the presence of a base in an inert solvent. As the base, 1 to 30 equivalents of lithium hydroxide, sodium hydroxide, potassium hydroxide, and the like may be used. As the solvent, aprotic solvents (for example, ether and tetrahydrofuran), protic solvents (for example, methanol and ethanol), water and the like may be used alone or in combination. The reaction is carried out at 10 to 120°C, and is completed in 5 to 100 hours.

Compounds which are the starting Compounds (III) in which at least one substituent within $R^4$, $R^5$, $R^6$ and $R^7$ is $-NR^8R^9$ can be alternatively prepared according to the following reaction steps.

Step 12

Compound (IIIb) which is Compound (III) in which at least one substituent within $R^4$, $R^5$, $R^6$ and $R^7$ is dimethylamino can be obtained by treating Compound (IIIa) which is Compound (III) in which at least one substituent within $R^4$, $R^5$, $R^6$ and $R^7$ is nitro in the presence of 2 to 1000 equivalents of formalin and a catalyst in an inert solvent under an atmosphere of hydrogen. As the catalyst, palladium, platinum and the like may be used alone or supported on activated carbon, barium sulfate or the like in an amount of 0.1 to 100 weight % of the amount of Compound (IIIa). As the solvent, aprotic solvents (for example, tetrahydrofuran and N,N-dimethylformamide), aromatic hydrocarbons (for example, toluene) and the like may be used alone or in combination. The reaction is carried out at 0°C to the boiling point of the solvent employed in the reaction, and is completed in 0.1 hour to 7 days.

Step 13

Compound (IIIc) which is Compound (III) in which at least one substituent within $R^4$, $R^5$, $R^6$ and $R^7$ is amino can be obtained by treating Compound (IIIa) in the presence of a reducing agent in an inert solvent. As the reducing agent, a sulfur compound (for example, sodium hydrosulfide and sodium bisulfite), a combination of hydrogen and a catalyst and the like may be used. As the catalyst, palladium, platinum and the like may be used alone or held on activated carbon, barium sulfate or the like in an amount of 0.1 to 100 weight % of the amount of Compound (IIIa). As the solvent, aprotic solvents (for example, tetrahydrofuran and N,N-dimethylformamide), protic solvents (for example, methanol and ethanol), aromatic hydrocarbons (for example, toluene), acetic acid, water and the like may be used alone or in combination. The reaction is carried out at 0°C to the boiling point of the solvent employed in the reaction, and is completed in 0.1 hour to 7 days.

Step 14

Compound (IIId) which is Compound (III) in which at least one substituent within $R^4$, $R^5$, $R^6$ and $R^7$ is $-NHCOR^{12a}$ (In the formula, $R^{12a}$ represents lower alkoxy. In the definition of $R^{12a}$, the lower alkoxy has the same meaning as defined above.) can be obtained by reacting Compound (IIIc) with Compound (XVI) represented by the formula $R^{12a}COY$ (In the formula, Y represents halogen, and $R^{12a}$ represents the same as defined above. In the definition of Y, the halogen has the same meaning as defined above.) in the presence of a base in an inert solvent. As the base, 1 to 100 equivalents of tertiary amines (for example, pyridine, triethylamine and diisopropylethylamine), inorganic bases (for example, sodium bicarbonate and potassium carbonate), metal hydrides (for example, sodium hydride) and the like may be used. As the solvent, aprotic solvents (for example, tetrahydrofuran and N,N-dimethylformamide), protic solvents (for example, methanol and ethanol), aromatic hydrocarbons (for example, toluene), halogenated hydrocarbons (for example, chloroform), water and the like may be used alone or in combination. The reaction is carried out at 0°C to the boiling point of the solvent employed in the reaction, and is completed in 0.1 hour to 3 days.

Step 15

Compound (IIIe) which is Compound (III) in which at least one substituent within $R^4$, $R^5$, $R^6$ and $R^7$ is $-NHCOR^{12b}$ (In the formula, $R^{12b}$ represents hydrogen, lower alkyl or substituted or unsubstituted aryl. In the definition of $R^{12b}$, the lower alkyl and substituted or unsubstituted aryl each have the same meanings as defined above.) can be obtained by reacting Compound (IIIc) with Compound (XVII) represented by the formula $R^{12b}COZ$ (In the formula, Z represents halogen or $R^{12b}CO_2$ wherein $R^{12b}$ has the same meaning as defined above. In the definition of Z, the halogen has the same meaning as defined above) according to the same method in Step 14.

Compound (IIIg) which is the starting Compound (III) in which $R^1$ is a group other than hydrogen and diglycolyl can be alternatively prepared according to the following reaction step.

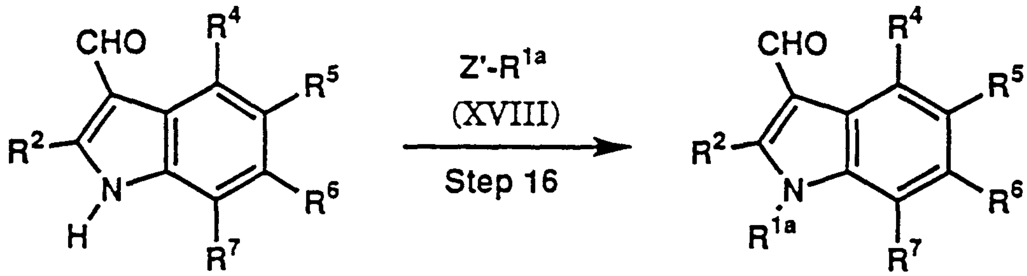


(IIIf) (IIIg)

(In the formulae, $R^{1a}$ represents a group other than hydrogen and diglycolyl in the definition of $R^1$, Z' represents halogen, p-nitrophenoxy or $R^{1aa}CO_2$ wherein $R^{1aa}$ represents lower alkyl or aralkyl in the definition of $R^{1a}$, and $R^2$, $R^4$, $R^5$, $R^6$ and $R^7$ have the same meanings as defined above. In the definition of Z', the halogen has the same meaning as defined above.)

Step 16

Compound (IIIg) can be obtained by reacting Compound (IIIf) which is Compound (III) in which $R^1$ is hydrogen with Compound (XVIII) according to the same method in Step 14.

Step 17

Compound (IIIh) which is Compound (III) in which $R^1$ is diglycolyl can be obtained according to the same method in Step 16, using diglycolic anhydride in place of Compound (XVIII).

Compound (Ib) which is Compound (I) in which $R^1$ is a group other than hydrogen and diglycolyl can be alternatively obtained according to the following reaction step.

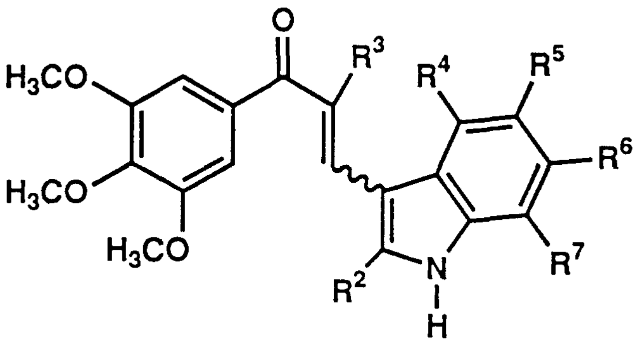

(Ia)

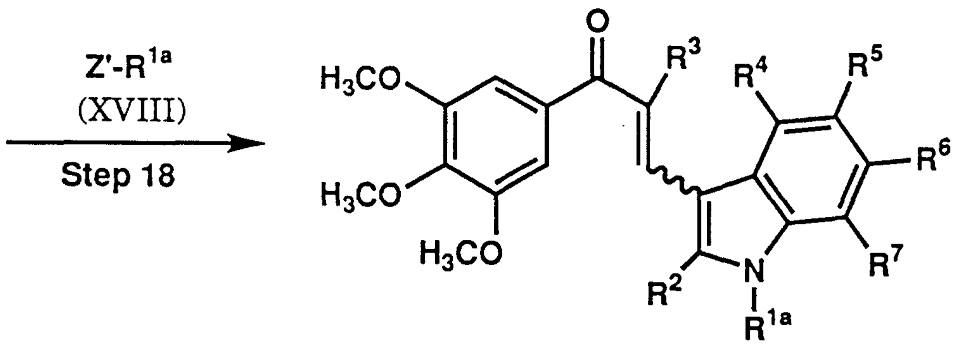


(Ib)

(In the formulae, $R^{1a}$, $R^2$, $R^3$, $R^4$, $R^5$, $R^6$, $R^7$ and Z' have the same meanings as defined above.)

Step 18

Compound (Ib) can be obtained by reacting Compound (Ia) which is Compound (I) in which $R^1$ is hydrogen with Compound (XVIII) according to the same method in Step 14.

Step 19

Compound (Ic) which is Compound (I) in which $R^1$ is diglycolyl can be obtained according to the same method in Step 18, using diglycolic anhydride in place of Compound (XVIII).

The intermediates and the desired compounds in the processes described above can be isolated and purified by purification methods conventionally used in organic synthetic chemistry, for example, filtration, extraction, washing, drying, concentration, recrystallization and various kinds of chromatography. The intermediates may also be subjected to the subsequent reaction without isolation.

In the case where a salt of Compound (I) is desired and it is produced in the form of the desired salt, it can be subjected to purification as such. In the case where Compound (I) is produced in the free form and its salt is desired, Compound (I) is dissolved or suspended in a suitable solvent, followed by addition of an acid to form a salt by a conventional method.

Compounds (I) can exist in the form of E/Z geometrical isomers, and the present invention covers all isomers including these geometrical isomers and mixtures thereof. In the case where Compound (I) is obtained in a E/Z mixture, and separation of E/Z isomers is desired, they can be isolated and purified by fractionation methods, for example, fractional crystallization, fractional precipitation, fractional dissolution or the like.

Compounds (I) and pharmaceutically acceptable salts thereof may be in the form of adducts with water or various solvents, which are also within the scope of the present invention.

Examples of Compounds (I) obtained in each process described above are shown in Table 1.

## Table 1-1

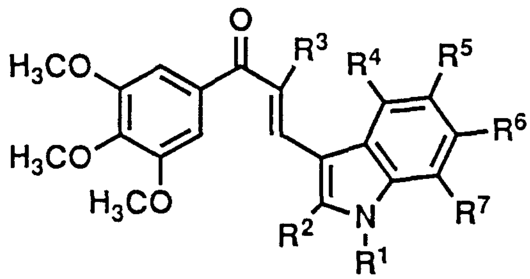

(I)

| Compd. No. | $R^1$ | $R^2$ | $R^3$ | $R^4$ | $R^5$ | $R^6$ | $R^7$ |
|---|---|---|---|---|---|---|---|
| 1 | H | H | H | H | H | H | H |
| 2 | $CH_3$ | H | H | H | H | H | H |
| 3 | $CH_3CH_2$ | H | H | H | H | H | H |
| 4 | $CH_3(CH_2)_3$ | H | H | H | H | H | H |
| 5 | $(CH_3)_2CHCH_2$ | H | H | H | H | H | H |
| 6 | $CH_3CO$ | H | H | H | H | H | H |
| 7 | $CH_3CH_2CO_2$ | H | H | H | H | H | H |
| 8 | $C_6H_5CO$ | H | H | H | H | H | H |
| 9 | $CH_3SO_2$ | H | H | H | H | H | H |
| 10 | $C_6H_5SO_2$ | H | H | H | H | H | H |
| 11 | DMDO [a] | H | H | H | H | H | H |
| 12 | H | $CH_3$ | H | H | H | H | H |
| 13 | $CH_3$ | Cl | H | H | H | H | H |
| 14 | H | $4\text{-}ClC_6H_4$ | H | H | H | H | H |
| 15 | H | H | H | $CH_3$ | H | H | H |
| 16 | $CH_3$ | H | H | $CH_3$ | H | H | H |
| 17 | H | H | H | $NO_2$ | H | H | H |
| 18 | H | H | H | $CH_3O$ | H | H | H |
| 19 | H | H | H | $CH_3O$ | H | H | $CH_3O$ |
| 20 | H | H | H | H | $CH_3$ | H | H |
| 21 | H | H | H | H | $NO_2$ | H | H |
| 22 | $CH_3$ | H | H | H | $(CH_3)_2N$ | H | H |
| 23 | $CH_3$ | H | H | H | $CH_3CH_2CO_2NH$ | H | H |
| 24 | $CH_3$ | H | H | H | $CH_3CONH$ | H | H |
| 25 | H | H | H | H | $CH_3O$ | H | H |

a)

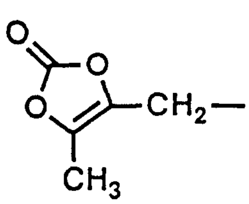

Table 1–2

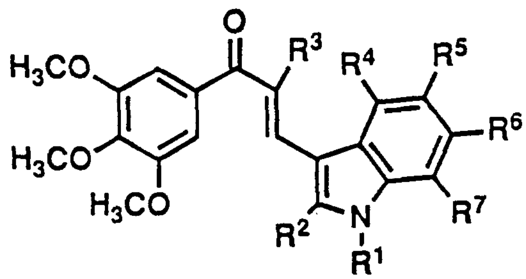

(I)

| Compd. No. | $R^1$ | $R^2$ | $R^3$ | $R^4$ | $R^5$ | $R^6$ | $R^7$ |
|---|---|---|---|---|---|---|---|
| 26 | H | H | H | H | $CH_3O$ | $CH_3O$ | H |
| 27 | H | H | H | H | OH | $CH_3O$ | H |
| 28 | H | H | H | H | $C_6H_5CH_2O$ | H | H |
| 29 | H | H | H | H | F | H | H |
| 30 | H | H | H | H | Cl | H | H |
| 31 | H | H | H | H | Br | H | H |
| 32 | H | H | H | H | H | $CH_3$ | H |
| 33 | H | H | H | H | H | $CF_3$ | H |
| 34 | H | H | H | H | H | $NO_2$ | H |
| 35 | $CH_3$ | H | H | H | H | $(CH_3)_2N$ | H |
| 36 | $CH_3$ | H | H | H | H | $CH_3CH_2OCONH$ | H |
| 37 | H | H | H | H | H | $CH_3CONH$ | H |
| 38 | $CH_3$ | H | H | H | H | $CH_3CONH$ | H |
| 39 | H | H | H | H | H | $CH_3O$ | H |
| 40 | H | H | H | H | H | $CH_3O$ | $CH_3O$ |
| 41 | H | H | H | H | H | OH | $CH_3O$ |
| 42 | H | H | H | H | H | Cl | H |
| 43 | H | H | H | H | H | H | $CH_3$ |
| 44 | H | H | H | H | H | H | $NO_2$ |
| 45 | $CH_3$ | H | H | H | H | H | $NO_2$ |
| 46 | H | H | H | H | H | H | $CH_3O$ |
| 47 | $C_6H_5CH_2$ | H | H | H | H | H | H |
| 48 | H | H | $CH_3$ | H | H | H | H |
| 49 | H | H | $CH_3(CH_2)_2$ | H | H | H | H |

Table 1–3

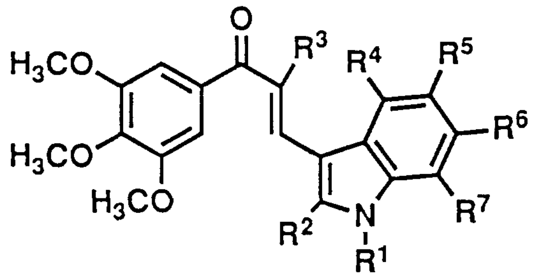

(I)

| Compd. No. | $R^1$ | $R^2$ | $R^3$ | $R^4$ | $R^5$ | $R^6$ | $R^7$ |
|---|---|---|---|---|---|---|---|
| 50 | $4\text{-}CH_3C_6H_4CO$ | H | H | H | H | H | H |
| 51 | $4\text{-}CH_3OC_6H_4CO$ | H | H | H | H | H | H |
| 52 | $3\text{-}CH_3C_6H_4CO$ | H | H | H | H | H | H |
| 53 | $3\text{-}CH_3OC_6H_4CO$ | H | H | H | H | H | H |
| 54 | $2\text{-}CH_3C_6H_4CO$ | H | H | H | H | H | H |
| 55 | $2\text{-}CH_3OC_6H_4CO$ | H | H | H | H | H | H |
| 56 | $4\text{-}CH_3CONHC_6H_4CO$ | H | H | H | H | H | H |
| 57 | $4\text{-}(CH_3)_2NC_6H_4CO$ | H | H | H | H | H | H |
| 58 | $3,4\text{-}(CH_3O)_2C_6H_3CO$ | H | H | H | H | H | H |
| 59 | $3,4,5\text{-}(CH_3O)_3C_6H_2CO$ | H | H | H | H | H | H |
| 60 | nicotinoyl | H | H | H | H | H | H |
| 61 | Isonicotinoyl | H | H | H | H | H | H |
| 62 | H | H | $CH_3CH_2$ | H | H | H | H |
| 63 | H | H | $CH_3$ | H | H | $CH_3$ | H |
| 64* | H | H | $C_6H_5$ | H | H | H | H |
| 65 | H | H | $CH_3(CH_2)_3$ | H | H | H | H |
| 66 | H | H | $CH_3$ | H | H | $CH_3CH_2$ | H |
| 67 | $HO_2CCH_2OCH_2CO$ | H | H | H | H | H | H |
| 68 | H | H | H | H | H | $(CH_3)_2CH$ | H |

* : a mixture of E-form and Z-form

The antitumor activities of Compounds (I) are shown in detail below by test examples.

Test Example 1: HeLa $S_3$ Cell Growth Inhibition Test

Each 0.1 ml of HeLa $S_3$ cells which had been prepared to 3 x $10^4$ cells/ml using a medium consisted of MEM medium, 10 % fetal bovine serum and 2 mM glutamine was distributed in each well of 96 well-microtiter plate. HeLa $S_3$ was cultured at 37°C in a $CO_2$ incubator for one night, each 0.05 ml of test compounds which had been appropriately diluted with the culture solution was added thereto and the mixture was cultured at 37°C for 72 hours in a $CO_2$ incubator. Supernatant was removed, each 0.1 ml of the culture solution containing 0.02 % neutral red was added to the residue, the mixture was incubated at 37°C for one hour in a $CO_2$ incubator and the cells were stained. Supernatant was removed and the residue was washed once with physiological saline. Then, the pigment was extracted with 0.001 N hydrochloric acid/30 % ethanol and the absorbance at 550 nm was measured by a microplatereader. A concentration of the test compound ($IC_{50}$) at which the growth of cell is inhibited by 50 % was calculated by comparing the absorbance of non-treated cells and that of cells treated with a predetermined concentration of the test compound.
The results are shown in Table 2.

Table 2

| Compd. No. | $IC_{50}$ (μM) | Compd. No. | $IC_{50}$ (μM) |
|---|---|---|---|
| 1 | 0.010 | 50 | 0.025 |
| 2 | 0.020 | 51 | 0.023 |
| 3 | 0.058 | 52 | 0.058 |
| 6 | 0.017 | 53 | 0.034 |
| 8 | 0.026 | 56 | 0.023 |
| 12 | 0.26 | 57 | 0.031 |
| 13 | 0.078 | 58 | 0.064 |
| 15 | 0.086 | 59 | 0.028 |
| 20 | 0.11 | 60 | 0.0077 |
| 29 | 0.060 | 61 | 0.0097 |
| 32 | 0.0063 | 62 | 0.0047 |
| 38 | 0.055 | 63 | 0.00073 |
| 40 | 0.012 | 64 | 0.0023 |
| 46 | 0.20 | 65 | 0.0063 |
| 48 | 0.00029 | 66 | 0.0025 |
| 49 | 0.0060 | 67 | 0.011 |

Test Example 2: Effect upon P388 Ascites Tumor

The experiment was carried out by using groups of 6-weeks-old male $CDF_1$ mice, each group consisting of five mice. $10^6$ cells of P388 mouse leukemia were implanted into the abdominal cavities of the mice. A test compound was sufficiently wetted by adding 10 μl of Tween 80 relative to 1 mg of a sample, and 0.3 % CMC (sodium carboxymethyl cellulose) solution was then added to the test compound to form a suspension. The resultant suspension was administered once 24 hours after implantation of the tumor, or repeatedly for consecutive 5 days from 24 hours after implantation of the tumor. The average survival day (T) in a group was calculated from the survival days of the respective mice in the group administered with the test compound at each dose. On the other hand, the average survival day (C) of a group which was not administered was measured, and the increased life span [ILS (%)] was calculated according to the following equation:

$$[(T - C)/C] \times 100 \ (\%)$$

The results are shown in Table 3.

Table 3

| Compd. No. | ILS(%)[Dose (mg/kg)] | |
|---|---|---|
| | single admin. | five consec. admin. |
| 1 | 94(200) | 113(100) |
| 3 | 35(50) | 56(100) |
| 8 | 33(100) | 75(100) |
| 32 | 44(100) | 53(50) |
| 40 | 25 (50) | 51 (100) |
| 48 | 61 (25) | 26 (2.0) |
| 49 | 39 (50) | 67 (4.0) |
| 51 | 24 (200) | 53 (100) |
| 56 | 13 (200) | 51 (100) |
| 63 | 48 (6.25) | 54 (1.25) |

The compounds obtained according to the present invention are useful as antitumor agents, and can be used as they are or in various administration forms. For example, when Compounds (I) are used as injections, Compounds

(I) may be dissolved in a diluting agent conventionally used in this field such as physiological saline, glucose injections, lactose injections, mannitol injections or the like, freeze-dried on the basis of the Japanese Pharmacopoeia, or mixed with sodium chloride to form powder injections. These injections may contain an adjuvant such as polyethylene glycol, HCO-60 (surfactant: produced by Nikko Chemical Co., Ltd.) or the like; and a carrier such as ethanol and/or liposome, cyclodextrin or the like. Although the injections are generally subjected to intravenous administration, they can also be subjected to arterial administration, intra-abdominal administration or intrathoracic administration.

When Compounds (I) are mixed with appropriate excipients, disintegrators, binders, lubricants and the like and formed to tablets, granules, powders, syrups or the like by a conventional method, the compounds can also be used as oral agents. Compounds (I) may be mixed with carriers conventionally used and formed, by a conventional method, to suppositories which can be administered to the rectum.

The dose varies depending upon the mode of administration, the type of Compound (I), the age and conditions of a patient, etc., and the administration schedule can be changed according to the conditions of a patient or the dose. For example, intravenous administration can be made at a dose of 0.01 to 100 mg/60 kg once a week or once every three weeks.

Examples are described below.

Best Mode for Carrying Out the Invention

The physicochemical data of each compound were measured by the following apparatus.

$^1$H-NMR: Nihon Denshi JNM-GX270 (270 MHz) Hitachi-90H (90 MHz)
MS: Nihon Denshi JSM-D300

Example 1

(E)-3-(Indol-3-yl)-1-(3,4,5-trimethoxyphenyl)-2-propen-1-one (Compound 1)

3',4',5'-Trimethoxyacetophenone (42.0 g) and indole-3-carboxaldehyde (29.0 g) were dissolved in ethanol (200 ml), and piperidine (17.0 g) was added thereto, followed by heating under reflux for 48 hours. The reaction solution was ice-cooled, and the precipitated crystals were collected by filtration to give Compound 1 (44.4 g).

$^1$H-NMR (270 MHz, DMSO-$d_6$) $\delta$ 3.78 (s, 3H), 3.92 (s, 6H), 7.19-7.28 (m, 2H), 7.36 (s, 2H), 7.49 (m, 1H), 7.61 (d, J = 15.4 Hz, 1H), 8.04 (m, 1H), 8.06 (d, J = 15.4 Hz, 1H), 8.13 (d, J = 3.0 Hz, 1H), 11.88 (brs, 1H)
EI-MS m/z = 337 ($M^+$)

Example 2

(E)-3-(1-Methylindol-3-yl)-1-(3,4,5-trimethoxyphenyl)-2-propen-1-one (Compound 2)

Compound 1 (1.0 g) obtained in Example 1 was dissolved in acetone (50 ml), and methyl iodide (1.42 g) and potassium carbonate (1.0 g) were added thereto, followed by heating under reflux for 72 hours. Insoluble matters were filtered off, and the filtrate was concentrated under reduced pressure. The residue was suspended in ethyl acetate (20 ml) in a condition of heating under reflux, and insoluble matters were filtered off. Hexane (25 ml) was added to the filtrate, and the precipitated crystals were collected by filtration to give Compound 2 (0.85 g).

$^1$H-NMR (270 MHz, DMSO-$d_6$) $\delta$ 3.77 (s, 3H), 3.87 (s, 3H), 3.92 (s, 6H), 7.21-7.34 (m, 2H), 7.36 (s, 2H), 7.48 (d, J = 7.8 Hz, 1H), 7.60 (d, J = 15.5 Hz, 1H), 8.00 (d, J = 15.5 Hz, 1H), 8.03 (d, J = 7.8 Hz, 1H), 8.12 (s, 1H)
EI-MS m/z = 351 ($M^+$)

Example 3

(E)-3-(1-Ethylindol-3-yl)-1-(3,4,5-trimethoxyphenyl)-2-propen-1-one (Compound 3)

Compound 1 (1.69 g) obtained in Example 1 was dissolved in a mixed solvent of N,N-dimethylformamide (12.5 ml) and tetrahydrofuran (12.5 ml), and ethyl bromide (817.3 mg) and sodium hydride (300 mg, 60 % mineral oil dispersion) were added thereto, followed by stirring at room temperature for 3 hours. Ice was added to the reaction solution, and the mixture was subjected to partitioning between ethyl acetate and water. The organic layer was dried over magnesium sulfate and concentrated under reduced pressure, and the residue was recrystallized from 2-propanol

to give Compound 3 (1.54 g).

$^1$H-NMR (270 MHz, $CDCl_3$) δ 1.54 (t, J = 7.3 Hz, 3H), 3.94 (s, 3H), 3.97 (s, 6H), 4.23 (q, J = 7.3 Hz, 2H), 7.31 (s, 2H), 7.34 (m, 2H), 7.41 (m, 1H), 7.48 (d, J = 15.5 Hz, 1H), 7.56 (s, 1H), 7.99 (m, 1H), 8.09 (d, J = 15.5 Hz, 1H)
EI-MS m/z = 365 ($M^+$)

Example 4

(E)-3-(1-Butylindol-3-yl)-1-(3,4,5-trimethoxyphenyl)-2-propen-1-one (Compound 4)

Substantially the same procedure as in Example 3 was repeated using Compound 1 (1.69 g) obtained in Example 1 and butyl bromide (0.81 ml) to give Compound 4 (1.48 g).

$^1$H-NMR (270 MHz, $CDCl_3$) δ 0.97 (t, J = 7.4 Hz, 3H), 1.38 (m, 2H), 1.87 (m, 2H), 3.94 (s, 3H), 3.97 (s, 6H), 4.17 (t, J = 7.4 Hz, 2H), 7.31 (s, 2H), 7.32 (m, 2H), 7.41 (m, 1H), 7.50 (d, J = 15.6 Hz, 1H), 7.52 (s, 1H), 7.99 (m, 1H), 8.09 (d, J = 15.6 Hz, 1H)
EI-MS m/z = 393 ($M^+$)

Example 5

(E)-3-(1-Isobutylindol-3-yl)-1-(3,4,5-trimethoxyphenyl)-2-propen-1-one (Compound 5)

Substantially the same procedure as in Example 3 was repeated using Compound 1 (1.69 g) obtained in Example 1 and isobutyl bromide (0.82 ml) to give Compound 5 (1.32 g).

$^1$H-NMR (270 MHz, $CDCl_3$) δ 0.97 (d, J = 6.9 Hz, 6H), 2.24 (m, 1H), 3.94 (s, 3H), 3.95 (d, J = 6.9 Hz, 2H), 3.97 (s, 6H), 7.30 (m, 2H), 7.31 (s, 2H), 7.35 (m, 1H), 7.48 (d, J = 15.3 Hz, 1H), 7.50 (s, 1H), 7.99 (m, 1H), 8.09 (d, J = 15.3 Hz, 1H)
EI-MS m/z = 393 ($M^+$)

Example 6

(E)-3-(1-Acetylindol-3-yl)-1-(3,4,5-trimethoxyphenyl)-2-propen-1-one (Compound 6)

A mixture of Compound 1 (1.69 g) obtained in Example 1, acetic anhydride (5.04 g) and pyridine (5.6 ml) was heated at 100°C for 12 hours. The reaction solution was subjected to partitioning between chloroform and a 10 % aqueous solution of citric acid. The organic layer was concentrated under reduced pressure, and the residue was recrystallized from ethyl acetate to give Compound 6 (1.32 g).

$^1$H-NMR (270 MHz, $CDCl_3$) δ 2.72 (s, 3H), 3.96 (s, 3H), 3.97 (s, 6H), 7.31 (s, 2H), 7.45 (m, 2H), 7.63 (d, J = 15.6 Hz, 1H), 7.81 (s, 1H), 7.93 (dd, J= 5.9, 1.9 Hz, 1H), 7.97 (d, J = 15.6 Hz, 1H), 8.51 (dd, J = 7.4, 1.9 Hz, 1H)
EI-MS m/z = 379 ($M^+$)

Example 7

(E)-3-(1-Ethoxycarbonylindol-3-yl)-1-(3,4,5-trimethoxyphenyl)-2-propen-1-one (Compound 7)

A mixture of Compound 1 (1.69 g) obtained in Example 1, ethoxycarbonyl chloride (2.5 ml) and pyridine (5 ml) was stirred at room temperature for 16 hours. The reaction solution was subjected to partitioning between chloroform and a 10 % aqueous solution of citric acid. The organic layer was concentrated under reduced pressure, and the residue was recrystallized from 2-propanol to give Compound 7 (1.83 g) .

$^1$H-NMR (270 MHz, $CDCl_3$) δ 1.51 (t, J = 7.2 Hz, 3H), 3.95 (s, 3H), 3.97 (s, 6H), 4.55 (q, J = 7.2 Hz, 2H), 7.30 (s, 2H), 7.42 (m, 2H), 7.60 (d, J = 15.8 Hz, 1H), 7.92 (m, 1H), 7.99 (d, J= 15.8 Hz, 1H), 8.04 (s, 1H), 8.26 (m, 1H)
EI-MS m/z = 409 ($M^+$)

Example 8

(E)-3-(1-Benzoylindol-3-yl)-1-(3,4,5-trimethoxyphenyl)-2-propen-1-one (Compound 8)

Substantially the same procedure as in Example 7 was repeated using Compound 1 (1.69 g) obtained in Example 1 and benzoyl chloride (1.16 ml) to give Compound 8 (0.91 g).

$^1$H-NMR (270 MHz, $CDCl_3$) $\delta$ 3.95 (s, 3H), 3.96 (s, 6H), 7.28 (s, 2H), 7.45-8.00 (m, 11H), 8.44 (m, 1H)
EI-MS m/z = 441 ($M^+$)

Example 9

(E)-3-(1-Methanesulfonylindol-3-yl)-1-(3,4,5-trimethoxyphenyl)-2-propen-1-one (Compound 9)

Substantially the same procedure as in Example 3 was repeated using Compound 1 (1.69 g) obtained in Example 1 and methanesulfonyl chloride (0.86 g) except that the reaction product was purified by silica gel column chromatography and that the obtained crude crystals were recrystallized from ethanol, to give Compound 9 (1.67 g).

$^1$H-NMR (270 MHz, $CDCl_3$) $\delta$ 3.22 (s, 3H), 3.95 (s, 3H), 3.97 (s, 6H), 7.30 (s, 2H), 7.47 (m, 2H), 7.60 (d, J = 15.5 Hz, 1H), 7.88 (s, 1H), 7.97 (m, 2H), 7.98 (d, J = 15.8 Hz, 1H)
EI-MS m/z = 415 ($M^+$)

Example 10

(E)-3-(1-Benzenesulfonylindol-3-yl)-1-(3,4,5-trimethoxyphenyl)-2-propen-1-one (Compound 10)

Substantially the same procedure as in Example 9 was repeated using Compound 1 (337.4 mg) obtained in Example 1 and benzenesulfonyl chloride (176.6 mg) to give Compound 10 (409.9 mg).

$^1$H-NMR (270 MHz, $CDCl_3$) $\delta$ 3.95 (s, 3H), 3.97 (s, 6H), 7.329 (s, 2H), 7.30-7.64 (m, 6H), 7.85-8.07 (m, 6H)
EI-MS m/z = 477 ($M^+$)

Example 11

(E)-3-[1-(4-Methyl-1,3-dioxol-2-on-5-yl)methylindol-3-yl]-1-(3,4,5-trimethoxyphenyl)-2-propen-1-one (Compound 11)

Substantially the same procedure as in Example 3 was repeated using Compound 1 (2.37 g) obtained in Example 1 and 4-chloromethyl-5-methyl-1,3-dioxol-2-one (1.63 g) except that the obtained crude crystals were further purified by preparative high-pressure liquid chromatography (HPLC), to give Compound 11 (188 mg).
EI-MS m/z = 449 ($M^+$)

Example 12

(E)-3-(2-Methylindol-3-yl)-1-(3,4,5-trimethoxyphenyl)-2-propen-1-one (Compound 12)

Substantially the same procedure as in Example 1 was repeated using 3',4',5'-trimethoxyacetophenone (2.10 g) and 2-methylindole-3-carboxaldehyde (1.59 g) except that the obtained crude crystals were recrystallized from ethanol, to give Compound 12 (2.13 g).

$^1$H-NMR (270 MHz, $CDCl_3$) $\delta$ 2.63 (s, 3H), 3.93 (s, 3H) , 3.97 (s, 6H), 7.22 (m, 2H), 7.32 (s, 2H), 7.38 (m, 1H), 7.47 (d, J = 15.3 Hz, 1H), 7.91 (dd, J = 6.4, 2.5 Hz, 1H), 8.17 (d, J = 15.3 Hz, 1H), 10.66 (brs, 1H)
EI-MS m/z = 351 ($M^+$)

Example 13

(E)-3-(2-Chloro-1-methylindol-3-yl)-1-(3,4,5-trimethoxyphenyl)-2-propen-1-one (Compound 13)

Process 1

3',4',5'-Trimethoxyacetophenone (15.0 g) and pyrrolidone hydrotribromide (35.39 g) were dissolved in tetrahydrofuran (225 ml), followed by stirring at 40°C for one hour. The reaction solution was cooled to room temperature, insoluble materials were filtered off, and the filtrate was concentrated under reduced pressure. The residue was purified by silica gel column chromatography, and the obtained crude crystals were washed with hexane (100 ml) to give 2-bromo-3',4',5'-trimethoxyacetophenone (Compound VII-1, 12.2 g) .

$^1$H-NMR (90 MHz, $CDCl_3$) δ 3.92 (s, 6H), 3.94 (s, 3H), 4.41 (s, 2H), 7.24 (s, 2H)
EI-MS m/z = 288, 290 ($M^+$)

Process 2

Compound VII-1 (12.12 g) obtained in the above Process 1 and triethyl phosphite (6.97 g) were dissolved in toluene (83 ml), followed by heating under reflux for 24 hours. The reaction solution was concentrated under reduced pressure, and then purified by silica gel column chromatography to give 2-diethylphosphono-3',4',5'-trimethoxyacetophenone (Compound IIc-1, 8.36 g).

$^1$H-NMR (90 MHz, $CDCl_3$) δ 1.30 (t, J = 7.0 Hz, 6H), 3.60 (d, J = 22.6 Hz, 2H), 3.92 (s, 9H), 4.14 (dq, J = 8.1, 7.0 Hz, 4H), 7.30 (s, 2H)
EI-MS m/z = 346 ($M^+$)

Process 3

2-Chloroindole-3-carboxaldehyde (2.69 g) and methyl iodide (4.26 g) were dissolved in a mixed solvent of tetrahydrofuran (25 ml) and dimethylsulfoxide (25 ml), and sodium hydride (0.66 g, 60 % mineral oil dispersion) was added thereto at 0°C. A 10 % aqueous solution of citric acid was added to the reaction solution, and the mixture was extracted with ethyl acetate. The organic layer was washed with water and then a saturated saline, and dried over anhydrous magnesium sulfate. After concentration under reduced pressure, the residue was washed with hexane (30 ml) to give 2-chloro-1-methylindole-3-carboxaldehyde (2.54 g).

$^1$H-NMR (90 MHz, $CDCl_3$) δ 3.80 (s, 3H), 7.29 (m, 3H), 8.29 (m, 1H), 10.11 (s, 1H)
EI-MS m/z = 193, 195 ($M^+$)

Process 4

Compound IIc-1 (692.6 mg) obtained in the above Process 2 was dissolved in tetrahydrofuran (136 ml), and potassium tert-butoxide (224.4 mg) and 2-chloro-1-methylindole-3-carboxaldehyde (287.2 mg) obtained in the above Process 3 were added thereto, followed by heating under reflux for 72 hours. The reaction solution was poured into ice-water, and the mixture was extracted with ethyl acetate. The organic layer was washed with water and then a saturated saline, and dried over anhydrous magnesium sulfate. After concentration under reduced pressure, the residue was purified by silica gel column chromatography, and the obtained crude crystals were recrystallized from 2-propanol to give Compound 13 (713.8 mg).

$^1$H-NMR (270 MHz, $CDCl_3$) δ 3.83 (s, 3H), 3.95 (s, 3H), 3.97 (s, 6H), 7.31 (s, 2H), 7.32 (m, 1H), 7.35 (m, 2H), 7.59 (d, J = 15.5 Hz, 1H), 7.93 (m, 1H), 8.12 (d, J = 15.5 Hz, 1H)
EI-MS m/z = 385 ($M^+$)

Example 14

(E)-3-[2-(4-Chlorophenyl)indol-3-yl]-1-(3,4,5-trimethoxyphenyl)-2-propen-1-one (Compound 14)

Substantially the same procedure as in Example 12 was repeated using 3',4',5'-trimethoxyacetophenone (0.84 g) and 2-(4-chlorophenyl)indole-3-carboxaldehyde (2.56 g) to give Compound 14 (3.04 g) .

$^1$H-NMR (270 MHz, $CDCl_3$) δ 3.94 (s, 9H), 7.29 (s, 2H), 7.34 (m, 2H), 7.45 (m, 1H), 7.46 (d, J = 9.9 Hz, 2H), 7.51 (d, J = 9.9 Hz, 2H), 7.63 (d, J = 15.3 Hz, 1H), 8.03 (m, 1H), 8.11 (d, J = 15.3 Hz, 1H), 8.80 (s, 1H)
EI-MS m/z = 447 ($M^+$)

Example 15

(E)-3-(4-Methylindol-3-yl)-1-(3,4,5-trimethoxyphenyl)-2-propen-1-one (Compound 15)

Process 1

A mixture of N,N-dimethylformamide (6 ml) and phosphorus oxychloride (1.78 g) was stirred at room temperature for 30 minutes. 4-Methylindole (1.0 g) was added to the reaction solution, followed by stirring at room temperature for one hour. A 5N aqueous solution of sodium hydroxide (15 ml) was added thereto, followed by stirring at 100°C for further 30 minutes. The reaction solution was cooled, and the precipitated crystals were collected by filtration to give 4-methylindole-3-carboxaldehyde (Compound III-1, 1.14 g) .

$^1$H-NMR (90 MHz, $CDCl_3$) δ 2.83 (s, 3H), 6.98-7.29 (m, 3H), 7.89 (d, J = 3.0 Hz, 1H), 9.11 (brs, 1H), 10.10 (s, 1H)
EI-MS m/z = 159 ($M^+$)

Process 2

Substantially the same procedure as in Example 1 was repeated using 3',4',5'-trimethoxyacetophenone (2.10 g) and Compound III-1 (2.56 g) obtained in the above Process 1 except that the obtained crude crystals were recrystallized from a mixed solvent of ethanol and acetone, to give Compound 15 (0.67 g).

$^1$H-NMR (270 MHz, $CDCl_3$) δ 2.80 (s, 3H), 3.94 (s, 3H), 3.95 (s, 6H), 6.99 (d, J = 7.4 Hz, 1H), 7.15 (t, J = 7.4 Hz, 1H), 7.295 (s, 2H), 7.295 (d, J = 16.1 Hz, 1H), 7.30 (d, J = 7.4 Hz, 1H), 7.80 (d, J = 3.0 Hz, 1H), 8.48 (d, J = 16.1 Hz, 1H), 8.63 (brs, 1H)
EI-MS m/z = 351 ($M^+$)

Example 16

(E)--3-(1,4-Dimethylindol-3-yl)-1-(3,4,5-trimethoxyphenyl)-2-propen-1-one (Compound 16)

Process 1

Compound III-1 (38 mg) obtained in Process 1 of Example 15 was reacted according to the same method in Example 2. The insoluble matters were filtered off, and the filtrate was concentrated under reduced pressure. The residue was subjected to partitioning between ethyl acetate and water, and the organic layer was dried over anhydrous magnesium sulfate and concentrated under reduced pressure to give 1,4-dimethylindole-3-carboxaldehyde (28.5 mg).

$^1$H-NMR (90 MHz, $CDCl_3$) δ 2.82 (s, 3H), 3.81 (s, 3H), 6.89-7.29 (m, 3H), 7.73 (s, 1H), 10.06 (s, 1H)
EI-MS m/z = 173 ($M^+$)

Process 2

Substantially the same procedure as in Process 4 of Example 13 was repeated using Compound IIc-1 (83 mg) obtained in Process 2 of Example 13 and 1,4-dimethylindole-3-carboxaldehyde (28 mg) obtained in the above Process 1 except that the reaction product was purified by silica gel column chromatography, to give Compound 16 (13.1 mg).

$^1$H-NMR (270 MHz, $CDCl_3$) δ 2.80 (s, 3H), 3.86 (s, 3H), 3.94 (s, 3H), 3.96 (s, 6H), 7.00 (m, 1H), 7.15-7.30 (m, 5H), 7.69 (s, 1H), 8.48 (d, J = 15.3 Hz, 1H)
EI-MS m/z = 365 ($M^+$)

Example 17

(E)-3-(4-Nitroindol-3-yl)-1-(3,4,5-trimethoxyphenyl)-2-propen-1-one (Compound 17)

Substantially the same procedure as in Example 1 was repeated using 3',4',5'-trimethoxyacetophenone (903 mg) and 4-nitroindole-3-carboxaldehyde (816 mg) to give Compound 17 (695 mg) .

$^1$H-NMR (270 MHz, DMSO-$d_6$) δ 3.83 (s, 3H), 3.91 (s, 6H), 7.37 (dd, J = 8.4, 7.9 Hz, 1H), 7.38 (s, 2H), 7.54 (d, J = 15.4 Hz, 1H), 7.91 (d, J= 8.4 Hz, 1H) , 7.92 (d, J = 7.9 Hz, 1H), 8.21 (d, J = 15.4 Hz, 1H), 8.65 (s, 1H), 12.66 (brs, 1H)
EI-MS m/z = 382 ($M^+$)

Example 18

(E)-3-(4-Methoxyindol-3-yl)-1-(3,4,5-trimethoxyphenyl)-2-propen-1-one (Compound 18)

Process 1

Substantially the same procedure as in Process 1 of Example 15 was repeated using 4-methoxyindole (1 g) to give 4-methoxyindole-3-carboxaldehyde (1.17 g).

$^1$H-NMR (90 MHz, $CDCl_3$) δ 3.97 (s, 3H), 6.52-7.35 (m, 4H), 8.24 (d, J = 4.8 Hz, 1H), 10.33 (s, 1H)
EI-MS m/z = 175 ($M^+$)

Process 2

Substantially the same procedure as in Example 12 was repeated using 3',4',5'-trimethoxyacetophenone (1.2 g) and 4-methoxyindole-3-carboxaldehyde (1 g) obtained in the above Process 1 to give Compound 18 (1.65 g).

$^1$H-NMR (270 MHz, $CDCl_3$) δ 3.77 (s, 3H), 3.91 (s, 6H), 3.95 (s, 3H), 6.67 (dd, J = 7.2, 1.3 Hz, 1H), 7.04-7:14 (m, 2H), 7.36 (s, 2H), 7.73 (d, J = 15.8 Hz, 1H), 8.24 (d, J= 3.0 Hz, 1H), 8.37 (d, J = 15.8 Hz, 1H), 8.68 (brs, 1H)
EI-MS m/z = 367 ($M^+$)

Example 19

(E)-3-(4,7-Dimethoxyindol-3-yl)-1-(3,4,5-trimethoxyphenyl)-2-propen-1-one (Compound 19)

Process 1

Substantially the same procedure as in Process 1 of Example 15 was repeated using 4,7-dimethoxyindole (0.78 g) to give 4,7-dimethoxyindole-3-carboxaldehyde (0.91 g).

$^1$H-NMR (90 MHz, $CDCl_3$) δ 3.92 (s, 6H), 6.58 (s, 2H), 7.89 (s, 1H), 10.48 (s, 1H)
EI-MS m/z = 205 ($M^+$)

Process 2

Substantially the same procedure as in Example 1 was repeated using 3',4',5'-trimethoxyacetophenone (1.05 g) and 4,7-dimethoxyindole-3-carboxaldehyde (0.75 g) obtained in the above Process 1 except that the reaction product was recrystallized from a mixed solvent of ethanol and water, to give Compound 19 (1.00 g).

$^1$H-NMR (270 MHz, $CDCl_3$) δ 3.93 (s, 3H), 3.94 (s, 3H), 3.94 (s, 3H), 3.95 (s, 6H), 6.50 (d, J = 8.4 Hz, 1H), 6.59 (d, J = 8.4 Hz, 2H), 7.30 (s, 2H), 7.57 (d, J = 15.8 Hz, 1H), 7.62 (d, J= 3.0 Hz, 1H), 8.38 (d, J = 15.8 Hz, 1H), 8.78 (brs, 1H)
EI-MS m/z = 397 ($M^+$)

Example 20

(E)-3-(5-Methylindol-3-yl)-1-(3,4,5-trimethoxyphenyl)-2-propen-1-one (Compound 20)

Substantially the same procedure as in Example 1 was repeated using 3',4',5'-trimethoxyacetophenone (2.1 g) and 5-methylindole-3-carboxaldehyde (1.59 g) except that the reaction solution was concentrated under reduced pressure, that the residue was purified by silica gel column chromatography, and that the obtained crude crystals were recrystallized from a mixed solvent of ethyl acetate and hexane, to give Compound 20 (1.16 g).

$^1$H-NMR (270 MHz, DMSO-$d_6$) δ 2.46 (s, 3H), 3.77 (s, 3H), 3.92 (s, 6H), 7.06 (d, J = 8.2 Hz, 1H), 7.36 (s, 2H), 7.37 (d, J = 8.2 Hz, 1H), 7.60 (d, J = 15.4 Hz, 1H), 7.82 (s, 1H), 8.02 (d, J= 15.4 Hz, 1H), 8.07 (d, J = 3.0 Hz, 1H), 11.77 (brs, 1H)
EI-MS m/z = 351 ($M^+$)

Example 21

(E)-3-(5-Nitroindol-3-yl)-1-(3,4,5-trimethoxyphenyl)-2-propen-1-one (Compound 21)

3',4',5'-Trimethoxyacetophenone (1.31 g) and 5-nitroindole-3-carboxaldehyde (1.18 g) were dissolved in N, N-dimethylformamide (20 ml), and piperidine (528.7 mg) was added thereto, followed by stirring at 100°C for 72 hours. The reaction solution was concentrated under reduced pressure, and the residue was purified by silica gel column chromatography. The obtained crude crystals were recrystallized from methanol to give Compound 21 (125 mg).

$^1$H-NMR (270 MHz, DMSO-$d_6$) δ 3.79 (s, 3H), 3.93 (s, 6H), 7.41 (s, 2H), 7.67 (d, J = 8.4 Hz, 1H), 7.79 (d, J = 15.6 Hz, 1H), 8.07 (d, J = 15.6 Hz, 1H), 8.11 (dd, J = 8.4, 2.0 Hz, 1H), 8.41 (s, 1H), 8.93 (d, J= 2.0 Hz, 1H), 12.48 (s, 1H)
CI-MS m/z = 382 (M)

Example 22

(E)-3-(5-Dimethylamino-1-methylindol-3-yl)-1-(3,4,5-trimethoxyphenyl)-2-propen-1-one (Compound 22)

Process 1

5-Nitroindole-3-carboxaldehyde (2 g) was dissolved in N,N-dimethylformamide (40 ml), and potassium carbonate (4.0 g) and methyl iodide (5 ml) were added thereto, followed by stirring at 60°C for 24 hours. The reaction solution was subjected to partitioning with chloroform, methanol and water, and the organic layer was dried over magnesium sulfate. After concentration under reduced pressure, the residue was rinsed with ethyl acetate to give 1-methyl-5-nitroindole-3-carboxaldehyde (Compound III-2, 1.99 g).

$^1$H-NMR (90 MHz, $CDCl_3$) δ 3.95 (s, 3H), 7.42 (d, J = 9.1 Hz, 1H), 7.83 (s, 1H), 8.23 (dd, J = 9.1, 2.2 Hz, 1H), 9.18 (d, J = 2.2 Hz, 1H), 10.04 (s, 1H)
EI-MS m/z = 204 ($M^+$)

Process 2

Compound III-2 (50 mg) obtained in the above Process 1 and 10 % palladium charcoal were suspended in a mixed solvent of methanol (20 ml) and N,N-dimethylformamide (5 ml), and. formalin (0.5 ml) was added thereto, followed by stirring at room temperature for 24 hours under an atmosphere of hydrogen. The catalyst was filtered off, and the filtrate was subjected to partitioning between chloroform and water. The organic layer was washed with water, dried over sodium sulfate and concentrated under reduced pressure to give 5-dimethylamino-1-methylindole-3-carboxaldehyde (45 mg) .

$^1$H-NMR (90 MHz, DMSO-$d_6$) δ 2.91 (s, 6H), 3.82 (s, 3H), 6.85 (m, 2H), 7.32 (m, 1H), 8.04 (s, 1H), 9.82 (s, 1H)
EI-MS m/z = 202 ($M^+$)

Process 3

5-Dimethylamino-1-methylindole-3-carboxaldehyde (45 mg) obtained in the above Process 2 and Compound

IId-1 (229 mg) obtained in Process 2 of Example 13 were dissolved in tetrahydrofuran (10 ml), and potassium tert-butoxide (74 mg) was added thereto, followed by stirring for 72 hours. The reaction solution was subjected to partitioning with chloroform, methanol and water, and the organic layer was dried over magnesium sulfate. After concentration under reduced pressure, the residue was purified by silica gel column chromatography and then medium-pressure silica gel column chromatography to give Compound 22 (17 mg).

$^1$H-NMR (270 MHz, DMSO-$d_6$) δ 2.97 (s, 6H), 3.78 (s, 3H), 3.81 (s, 3H), 3.91 (s, 6H), 6.91 (dd, J = 9.1, 2.2 Hz, 1H), 7.21 (d, J = 2.2 Hz, 1H), 7.36 (s, 2H), 7.39 (d, J = 9.1 Hz, 1H), 7.54 (d, J = 15.4 Hz, 1H), 7.96 (s, 1H), 7.99 (d, J = 15.4 Hz, 1H)
EI-MS m/z = 394 ($M^+$)

Example 23

(E)-3-(5-Ethoxycarbonylamino-1-methylindol-3-yl)-1-(3,4,5-trimethoxyphenyl)-2-propen-1-one (Compound 23)

Process 1

Compound III-2 (200 mg) obtained in Process 1 of Example 22 was suspended in a mixed solvent of methanol (25 ml) and water (25 ml), and sodium hydrosulfite (1.0 g) was added thereto, followed by heating under reflux for one hour.

Process 2

A 5 % aqueous solution of sodium bicarbonate (20 ml) was added to the reaction solution obtained in the above Process 1, and ethyl chloroformate (125 mg) was added thereto, followed by stirring at room temperature for 24 hours. The reaction solution was subjected to partitioning between chloroform and water, and the organic layer was dried over magnesium sulfate. After concentration under reduced pressure, the residue was purified by silica gel column chromatography to give 5-ethoxycarbonylamino-1-methylindole-3-carboxaldehyde (38 mg) .

$^1$H-NMR (90 MHz, DMSO-$d_6$) δ 1.26 (t, J = 7.0 Hz, 3H), 3.85 (s, 3H), 4.14 (q, J = 7.0 Hz, 1H), 7.42 (s, 2H), 8.15 (s, 1H), 8.27 (s, 1H), 9.67 (s, 1H), 9.84 (s, 1H)
EI-MS m/z = 246 ($M^+$)

Process 3

Substantially the same procedure as in Process 3 of Example 22 was repeated using 5-ethoxycarbonylamino-1-methylindole-3-carboxaldehyde (20 mg) obtained in the above Process 2 and Compound IIc-1 (84 mg) obtained in Process 2 of Example 13 except that the residue was purified by silica gel column chromatography and that the obtained crude crystals were rinsed with ethyl acetate, to give Compound 23 (17 mg).

$^1$H-NMR (270 MHz, DMSO-$d_6$) δ 1.29 (t, J = 7.1 Hz, 3H), 3.78 (s, 3H), 3.83 (s, 3H), 3.95 (s, 6H), 4.15 (q, J = 7.1 Hz, 2H), 7.26 (dd, J = 8.9, 2.0 Hz, 1H), 7.35 (s, 2H), 7.46 (d, J = 8.9 Hz, 1H), 7.56 (d, J = 15.3 Hz, 1H), 7.97 (d, J = 15.3 Hz, 1H), 8.02 (s, 1H), 8.49 (s, 1H), 9.63 (s, 1H)
EI-MS m/z = 438 ($M^+$)

Example 24

(E)-3-(5-Acetamido-1-methylindol-3-yl)-1-(3,4,5-trimethoxyphenyl)-2-propen-1-one (Compound 24)

Process 1

Substantially the same procedure as in Process 1 of Example 23 was repeated using Compound III-2 (200 mg) obtained in Process 1 of Example 22. Pyridine (5 ml) and acetic anhydride (5 ml) were added to the reaction solution, followed by stirring at room temperature for 24 hours. The reaction solution was subjected to partitioning between the organic layer was dried over magnesium sulfate. After concentration under reduced pressure, the residue was suspended in toluene, and the mixture was concentrated again under reduced pressure to give 5-acetamido-1-methylindole-3-carboxaldehyde (53 mg).

$^1$H-NMR (270 MHz, DMSO-$d_6$) δ 2.16 (s, 3H), 3.87 (s, 3H), 7.30 (d, J = 8.9 Hz, 1H), 7.71 (s, 1H), 7.92 (dd, J = 8.9, 1.9 Hz, 1H), 8.18 (d, J = 1.9 Hz, 1H), 9.18 (brs, 1H), 9.94 (s, 1H)
EI-MS m/z = 216 ($M^+$)

Process 2

Substantially the same procedure as in Process 3 of Example 23 was repeated using 5-acetamido-1-methylindole-3-carboxaldehyde (37 mg) obtained in the above Process 1 and Compound IIc-1 (118 mg) obtained in Process 2 of Example 13 to give Compound 24 (37 mg).

$^1$H-NMR (270 MHz, DMSO-$d_6$) δ 2.08 (s, 3H), 3.77 (s, 3H), 3.84 (s, 3H), 3.94 (s, 6H), 7.32 (dd, J = 8.9, 1.5 Hz, 1H), 7.39 (s, 2H), 7.48 (d, J = 8.9 Hz, 1H), 7.56 (d, J = 15.8 Hz, 1H), 7.96 (d, J = 15.8 Hz, 1H), 8.04 (s, 1H), 8.63 (d, J = 1.5 Hz, 1H), 9.97 (s, 1H)
FAB-MS m/z = 409 ($M^+$ + 1)

Example 25

(E)-3-(5-Methoxyindol-3-yl)-1-(3,4,5-trimethoxyphenyl)-2-propen-1-one (Compound 25)

Substantially the same procedure as in Example 1 was repeated using 3',4',5'-trimethoxyacetophenone (1.2 g) and 5-methoxyindole-3-carboxaldehyde (1 g) except that the obtained crude crystals were recrystallized from 2-propanol, to give Compound 25 (1.09 g).

$^1$H-NMR (270 MHz, DMSO-$d_6$) δ 3.77 (s, 3H), 3.87 (s, 3H), 3.91 (s, 6H), 6.86 (dd, J = 8.7, 2.3 Hz, 1H), 7.38 (d, J = 8.7 Hz, 1H), 7.39 (s, 2H), 7.47 (d, J = 2.3 Hz, 1H), 7.58 (d, J = 15.5 Hz, 1H), 7.93 (d, J = 2.5 Hz, 1H), 8.01 (d, J = 15.5 Hz, 1H), 8.68 (s, 1H)
EI-MS m/z = 397 ($M^+$)

Example 26

(E)-3-(5,6-Dimethoxyindol-3-yl)-1-(3,4,5-trimethoxyphenyl)-2-propen-1-one (Compound 26)

Process 1

Substantially the same procedure as in Process 1 of Example 15 was repeated using 5,6-dimethoxyindole (1 g) to give 5,6-dimethoxyindole-3-carboxaldehyde (914 mg).

$^1$H-NMR (90 MHz, DMSO-$d_6$) δ 3.81 (s, 6H), 7.02 (s, 1H), 7.57 (s, 1H), 8.02 (d, J = 2.9 Hz, 1H), 9.85 (s, 1H), 11.77 (brs, 1H)
EI-MS m/z = 205 ($M^+$)

Process 2

Substantially the same procedure as in Example 1 was repeated using 3',4',5'-trimethoxyacetophenone (834 mg) and 5,6-dimethoxyindole-3-carboxaldehyde (814 mg) obtained in the above Process 1 except that the obtained crude crystals were recrystallized from a mixed solvent of 2-propanol and acetone, to give Compound 26 (900 mg).

$^1$H-NMR (270 MHz, DMSO-$d_6$) δ 3.77 (s, 3H), 3.82 (s, 3H), 3.90 (s, 3H), 3.91 (s, 6H), 7.00 (s, 1H), 7.39 (s, 2H), 7.48 (s, 1H), 7.58 (d, J = 15.5 Hz, 1H), 7.93 (d, J = 2.5 Hz, 1H), 8.01 (d, J = 15.5 Hz, 1H), 8.68 (s, 1H)
EI-MS m/z = 397 ($M^+$)

Example 27

(E)-3-(5-Hydroxy-6-methoxyindol-3-yl)-1-(3,4,5-trimethoxyphenyl)-2-propen-1-one (Compound 27)

Process 1

Substantially the same procedure as in Process 1 of Example 15 was repeated using 5-benzyloxy-6-methoxyindole (0.85 g) to give 5-benzyloxy-6-methoxyindole-3-carboxaldehyde (0.79 g).

$^1$H-NMR (90 MHz, $CDCl_3$) δ 3.90 (s, 3H), 5.20 (s, 2H), 6.91 (s, 1H), 7.25-7.55 (s, 5H), 7.66 (d, J = 3.3 Hz, 1H), 7.88 (s, 1H), 8.59 (brs, 1H), 9.97 (s, 1H)
EI-MS m/z = 281 ($M^+$)

Process 2

5-Benzyloxy-6-methoxyindole-3-carboxaldehyde (0.75 g) was suspended in a mixed solvent of methanol (60 ml) and ethyl acetate (20 ml), and 10 % palladium on carbon (85 mg) was added thereto, followed by stirring at room temperature for one hour under an atmosphere of hydrogen. The catalyst was filtered off, and the filtrate was concentrated under reduced pressure to give 5-hydroxy-6-methoxyindole-3-carboxaldehyde (0.51 g).

$^1$H-NMR (90 MHz, $CDCl_3$) δ 3.89 (s, 3H), 6.95 (s, 1H), 7.62 (s, 1H), 7.74 (d, J = 3.1 Hz, 1H), 8.17 (s, 1H), 9.86 (s, 1H), 11.43 (s, 1H)
EI-MS m/z = 191 ($M^+$)

Process 3

Substantially the same procedure as in Example 12 was repeated using 3',4',5'-trimethoxyacetophenone (0.55 g) and 5-hydroxy-6-methoxyindole-3-carboxaldehyde (0.51 g) obtained in the above Process 2 to give Compound 27 (93 mg) .

$^1$H-NMR (270 MHz, $CDCl_3$) δ 3.94 (s, 3H), 3.95 (s, 3H), 3.96 (s, 6H), 5.62 (s, 1H), 6.92 (s, 1H), 7.31 (s, 2H), 7.43 (d, J = 15.5 Hz, 1H), 7.49 (d, J = 3.0 Hz, 1H), 7.50 (s, 1H), 8.05 (d, J = 15.5 Hz, 1H), 8.53 (s, 1H)
EI-MS m/z = 383 ($M^+$)

Example 28

(E)-3-(5-Benzyloxyindol-3-yl)-1-(3,4,5-trimethoxyphenyl)-2-propen-1-one (Compound 28)

Substantially the same procedure as in Example 1 was repeated using 3',4',5'-trimethoxyacetophenone (2.1 g) and 5-benzyloxyindole-3-carboxaldehyde (2.51 g) except that the obtained crude crystals were recrystallized twice from acetone, to give Compound 28 (107 mg) .

$^1$H-NMR (270 MHz, $CDCl_3$) δ 3.942 (s, 3H), 3.947 (s, 6H), 5.16 (s, 2H), 7.04 (dd, J = 7.9, 2.2 Hz, 1H), 7.29 (s, 2H), 7.31-7.55 (m, 8H), 7.61 (d, J = 2.9 Hz, 1H), 8.08 (d, J = 15.7 Hz, 1H), 8.53 (s, 1H)
EI-MS m/z = 443 ($M^+$)

Example 29

(E)-3-(5-Fluoroindol-3-yl)-1-(3,4,5-trimethoxyphenyl)-2-propen-1-one (Compound 29)

Substantially the same procedure as in Example 12 was repeated using 3',4',5'-trimethoxyacetophenone (2.1 g) and 5-fluoroindole-3-carboxaldehyde (1.63 g) to give Compound 29 (2.1 g).

$^1$H-NMR (270 MHz, $CDCl_3$) δ 3.95 (s, 3H), 3.97 (s, 6H), 7.07 (td, J = 8.9, 2.5 Hz, 1H), 7.31 (s, 2H), 7.39 (dd, J = 8.9, 4.5 Hz, 1H), 7.44 (d, J = 15.5 Hz, 1H), 7.62 (dd, J = 9.4, 2.5 Hz, 1H), 7.66 (d, J = 3.0 Hz, 1H), 8.05 (d, J = 15.5 Hz, 1H), 8.82 (brs, 1H)
EI-MS m/z = 355 ($M^+$)

Example 30

(E)-3-(5-Chloroindol-3-yl)-1-(3,4,5-trimethoxyphenyl)-2-propen-1-one (Compound 30)

Substantially the same procedure as in Example 12 was repeated using 3',4',5'-trimethoxyacetophenone (2.1 g) and 5-chloroindole-3-carboxaldehyde (1.8 g) to give Compound 30 (2.94 g).

$^1H$-NMR (270 MHz, DMSO-$d_6$) δ 3.77 (s, 3H), 3.92 (s, 6H), 7.23 (dd, J = 8.5, 2.0 Hz, 1H), 7.38 (s, 2H), 7.50 (d, J = 8.5 Hz, 1H), 7.63 (d, J = 15.5 Hz, 1H), 8.00 (d, J = 15.5 Hz, 1H), 8.07 (d, J = 2.0 Hz, 1H), 8.22 (s, 1H), 12.05 (brs, 1H)
EI-MS m/z = 371, 373 ($M^+$)

Example 31

(E)-3-(5-Bromoindol-3-yl)-1-(3,4,5-trimethoxyphenyl)-2-propen-1-one (Compound 31)

Substantially the same procedure as in Example 12 was repeated using 3',4',5'-trimethoxyacetophenone (2.1 g) and 5-bromoindole-3-carboxaldehyde (2.24 g) except that the obtained crystals were recrystallized from ethyl acetate, to give Compound 31 (2.73 g).

$^1H$-NMR (270 MHz, DMSO-$d_6$) δ 3.78 (s, 3H), 3.92 (s, 6H), 7.34 (dd, J = 8.8, 2.0 Hz, 1H), 7.38 (s, 2H), 7.46 (d, J = 8.8 Hz, 1H), 7.63 (d, J = 15.3 Hz, 1H), 8.00 (d, J = 15.3 Hz, 1H), 8.19 (d, J = 2.0 Hz, 1H), 8.20 (s, 1H), 12.04 (brs, 1H)
EI-MS m/z = 415, 417 ($M^+$)

Example 32

(E)-3-(6-Methylindol-3-yl)-1-(3,4,5-trimethoxyphenyl)-2-propen-1-one (Compound 32)

Process 1

Substantially the same procedure as in Process 1 of Example 15 was repeated using 6-methylindole (1.15 g) to give 6-methylindole-3-carboxaldehyde (1.34 g).

$^1H$-NMR (270 MHz, $CDCl_3$) δ 2.47 (s, 3H), 7.10 (d, J = 8.0 Hz, 1H), 7.24 (brs, 1H), 7.75 (d, J = 2.6 Hz, 1H), 8.18 (d, J = 8.0 Hz, 1H), 9.99 (s, 1H), 10.55 (brs, 1H)
EI-MS m/z = 159 ($M^+$)

Process 2

Substantially the same procedure as in Example 12 was repeated using 3',4',5'-trimethoxyacetophenone (1.05 g) and 6-methylindole-3-carboxaldehyde (0.8 g) obtained in the above Process 1 to give Compound 32 (0.77 g).

$^1H$-NMR (270 MHz, $CDCl_3$) δ 2.49 (s, 3H), 3.94 (s, 3H), 3.96 (s, 6H), 7.14 (d, J = 8.2 Hz, 1H), 7.25 (s, 1H), 7.31 (s, 2H), 7.50 (d, J = 15.5 Hz, 1H), 7.56 (d, J = 3.0 Hz, 1H), 7.87 (d, J = 8.2 Hz, 1H), 8.08 (d, J = 15.5 Hz, 1H), 8.68 (brs, 1H)
EI-MS m/z = 351 ($M^+$)

Example 33

(E)-3-(6-Trifluoromethylindol-3-yl)-1-(3,4,5-trimethoxyphenyl)-2-propen-1-one (Compound 33)

Process 1

Substantially the same procedure as in Process 1 of Example 15 was repeated using 6-trifluoromethylindole (1.85 g) to give 6-trifluoromethylindole-3-carboxaldehyde (1.90 g).

$^1H$-NMR (90 MHz, $CDCl_3$) δ 7.44 (brd, J = 8.4 Hz, 1H) , 7.73 (brs, 1H), 7.80 (d, J = 3.0 Hz, 1H), 8.33 (d, J = 8.4 Hz, 1H), 10.03 (s, 1H)
EI-MS m/z = 213 ($M^+$)

Process 2

Substantially the same procedure as in Example 12 was repeated using 3',4',5'-trimethoxyacetophenone (2.1 g) and 6-trifluoromethylindole-3-carboxaldehyde (1.67 g) obtained in the above Process 1 to give Compound 33 (1.38 g).

$^1$H-NMR (270 MHz, $CDCl_3$) δ 3.95 (s, 3H), 3.97 (s, 6H), 7.31 (s, 2H), 7.53 (d, J = 15.8 Hz, 1H), 7.54 (brd, J = 8.4 Hz, 1H), 7.75 (brs, 1H), 7.78 (d, J = 3.0 Hz, 1H), 8.06 (d, J = 8.4 Hz, 1H), 8.09 (d, J = 15.8 Hz, 1H), 9.02 (brs, 1H)
EI-MS m/z = 405 ($M^+$)

Example 34

(E)-3-(6-Nitroindol-3-yl)-1-(3,4,5-trimethoxyphenyl)-2-propen-1-one (Compound 34)

Substantially the same procedure as in Example 21 was repeated using 3',4',5'-trimethoxyacetophenone (1.25 g) and 6-nitroindole-3-carboxaldehyde (1.13 g) except that the obtained product was further purified by preparative HPLC, that the eluting solution was cooled, and that the precipitated crystals were collected by filtration, to give Compound 34 (26 mg).

$^1$H-NMR (270 MHz, DMSO-$d_6$) δ 3.78 (s, 3H), 3.92 (s, 6H), 7.39 (s, 2H), 7.76 (d, J = 15.9 Hz, 1H), 8.055 (dd, J = 8.9, 2.0 Hz, 1H), 8.056 (d, J = 15.9 Hz, 1H), 8.25 (d, J = 8.9 Hz, 1H), 8.41 (d, J = 2.0 Hz, 1H), 8.54 (s, 1H), 12.51 (brs, 1H)
CI-MS m/z = 382 (M)

Example 35

(E)-3-(6-Dimethyl-1-methylindol-3-yl)-1-(3,4,5-trimethoxyphenyl)-2-propen-1-one (Compound 35)

Process 1

Substantially the same procedure as in Process 1 of Example 22 was repeated using 6-nitroindole-3-carboxaldehyde (2 g) to give 1-methyl-6-nitroindole-3-carboxaldehyde (Compound III-3, 1.91 g).

$^1$H-NMR (270 MHz, DMSO-$d_6$) δ 4.04 (s, 3H), 8.12 (dd, J = 8.2, 2.0 Hz, 1H), 8.29 (d, J = 8.2 Hz, 1H), 8.32 (s, 1H), 8.43 (d, J = 2.0 Hz, 1H)
EI-MS m/z = 204 ($M^+$)

Process 2

Substantially the same procedure as in Process 2 of Example 22 was repeated using Compound III-3 (350 mg) obtained in the above Process 1 to give 6-dimethylamino-1-methylindole-3-carboxaldehyde (203 mg).

$^1$H-NMR (90 MHz, DMSO-$d_6$) δ 2.96 (s, 6H), 3.81 (s, 3H), 6.70 (d, J = 1.8 Hz, 1H), 6.80 (dd, J = 8.8, 1.8 Hz, 1H), 7.88 (d, J = 8.8 Hz, 1H), 7.97 (s, 1H), 9.78 (s, 1H)
EI-MS m/z = 202 ($M^+$)

Process 3

Substantially the same procedure as in Example 20 was repeated using 3',4',5'-trimethoxyacetophenone (184 mg) and 6-dimethylamino-1-methylindole-3-carboxaldehyde (177 mg) obtained in the above Process 2 except that the obtained crude crystals were purified by preparative HPLC, to give Compound 35 (110 mg) .

$^1$H-NMR (270 MHz, DMSO-$d_6$) δ 2.98 (s, 6H), 3.77 (s, 3H), 3.78 (s, 3H), 3.91 (s, 6H), 6.72 (d, J = 2.3 Hz, 1H), 6.84 (dd, J = 8.9, 2.3 Hz, 1H), 7.33 (s, 2H), 7.53 (d, J = 15.3 Hz, 1H), 7.84 (s, 1H), 7.88 (d, J = 8.9 Hz, 1H), 7.93 (d, J = 15.3 Hz, 1H)
EI-MS m/z = 394 ($M^+$)

Example 36

(E)-3-(6-Ethoxycarbonylamino-1-methylindol-3-yl)-1-(3,4,5-trimethoxyphenyl)-2-propen-1-one (Compound 36)

Process 1

Substantially the same procedure as in Processes 1 and 2 of Example 23 was repeated using Compound III-3 (200 mg) obtained in Process 1 of Example 35 to give 6-ethoxycarbonylamino-1-methylindole-3-carboxaldehyde (38 mg).

$^1$H-NMR (90 MHz, DMSO-$d_6$) δ 1.27 (d, J = 7.0 Hz, 3H), 3.82 (s, 3H), 4.15 (q, J = 7.0 Hz, 2H), 7.21 (dd, J = 8.5, 1.8 Hz, 1H), 7.81 (d, J = 1.8 Hz, 1H), 7.94 (d, J = 8.5 Hz, 1H), 8.13 (s, 1H), 9.64 (s, 1H), 9.78 (s, 1H)
EI-MS m/z = 246 ($M^+$)

Process 2

Substantially the same procedure as in Process 3 of Example 23 was repeated using 6-ethoxycarbonylamino-1-methylindole-3-carboxaldehyde (20 mg) obtained in the above Process 1 and Compound II-c (84 mg) obtained in Process 2 of Example 13 to give Compound 36 (17 mg).

$^1$H-NMR (270 MHz, DMSO-$d_6$) δ 1.28 (t, J = 7.0 Hz, 3H), 3.77 (s, 3H), 3.79 (s, 3H), 3.91 (s, 6H), 4.16 (q, J = 7.0 Hz, 1H), 7.23 (dd, J = 8.5, 1.3 Hz, 1H), 7.35 (s, 2H), 7.54 (d, J = 15.3 Hz, 1H), 7.81 (s, 1H), 7.94 (d, J = 15.3 Hz, 1H), 7.96 (d, J = 8.5 Hz, 1H), 8.01 (s, 1H), 9.71 (s, 1H)
EI-MS m/z = 438 ($M^+$)

<u>Example 37</u>

(E)-3-(6-Acetamidoindol-3-yl)-1-(3,4,5-trimethoxyphenyl)-2-propen-1-one (Compound 37)

Process 1

Substantially the same procedure as in Example 3 was repeated using 6-nitroindole-3-carboxaldehyde (2.3 g) and acetic anhydride (2.47 g) to give 1-acetyl-6-nitroindole-3-carboxaldehyde (2.62 g) .

$^1$H-NMR (90 MHz, DMSO-$d_6$) δ 2.81 (s, 3H), 8.31 (s, 2H), 9.20 (s, 2H), 10.13 (s, 1H)
EI-MS m/z = 232 ($M^+$)

Process 2

Substantially the same procedure as in Process 1 of Example 24 was repeated using 1-acetyl-6-nitroindole-3-carboxaldehyde (100 mg) obtained in the above Process 1 to give 6-acetamidoindole-3-carboxaldehyde (28 mg) .

$^1$H-NMR (90 MHz, DMSO-$d_6$) δ 2.08 (s, 3H), 7.18 (dd, J = 8.4, 1.7 Hz, 1H), 7.95 (d, J = 8.4 Hz, 1H), 8.10 (d, J = 1.7 Hz, 1H), 8.16 (d, J = 2.8 Hz, 1H), 9.88 (s, 1H), 9.93 (brs, 1H), 11.95 (brs, 1H)
EI-MS m/z = 202 ($M^+$)

Process 3

Substantially the same procedure as in Example 1 was repeated using 3',4',5'-trimethoxyacetophenone (252 mg) and 6-acetamidoindole-3-carboxaldehyde (243 mg) obtained in the above Process 2 except that the obtained crude crystals were recrystallized from a mixed solvent of ethanol, acetone and water, to give Compound 37 (98 mg).

$^1$H-NMR (270 MHz, DMSO-$d_6$) δ 2.08 (s, 3H), 3.77 (s, 3H), 3.87 (s, 6H), 7.23 (dd, J = 8.6, 1.5 Hz, 1H), 7.35 (s, 2H), 7 55 (d, J = 15.5 Hz, 1H), 7.95 (d, J = 8.6 Hz, 1H), 7.99 (d, J = 15.5 Hz, 1H), 8.03 (d, J = 3.0 Hz, 1H), 8.08 (d, J = 1.5 Hz, 1H), 9.96 (s, 1H), 11.77 (brs, 1H)
EI-MS m/z = 394 ($M^+$)

<u>Example 38</u>

(E)-3-(6-Acetamido-1-methylindol-3-yl)-1-(3,4,5-trimethoxyphenyl)-2-propen-1-one (Compound 38)

Process 1

Substantially the same procedure as in Process 1 of Example 24 was repeated using Compound III-3 (200 mg) obtained in Process 1 of Example 35 to give 6-acetamido-1-methylindole-3-carboxaldehyde (68 mg).

$^1$H-NMR (90 MHz, DMSO-$d_6$) δ 2.18 (s, 3H), 3.85 (s, 3H), 7.12 (dd, J = 8.7, 1.6 Hz, 1H), 7.70 (s, 1H), 8.11 (d, J = 8.7 Hz, 1H), 8.19 (d, J = 1.6 Hz, 1H), 8.16 (d, J = 2.8 Hz, 1H), 9.30 (brs, 1H), 9.92 (brs, 1H)
EI-MS m/z = 216 ($M^+$)

Process 2

Substantially the same procedure as in Process 3 of Example 23 was repeated using 6-acetamido-1-methylindole-3-carboxaldehyde (51 mg) obtained in the above Process 1 and Compound IIc-1 (245 mg) obtained in Process 2 of Example 13 to give Compound 38 (52 mg).

$^1$H-NMR (270 MHz, DMSO-$d_6$) δ 2.09 (s, 3H), 3.77 (s, 3H), 3.80 (s, 3H), 3.92 (s, 6H), 7.29 (dd, J = 8.4, 1.5 Hz, 1H), 7.35 (s, 2H), 7.55 (d, J = 15.3 Hz, 1H), 7.95 (d, J = 15.3 Hz, 1H), 7.98 (d, J = 8.4 Hz, 1H), 8.01 (d, J = 1.5 Hz, 1H), 8.02 (s, 1H), 10.03 (s, 1H)
EI-MS m/z = 408 ($M^+$)

Example 39

(E)-3-(6-Methoxyindol-3-yl)-1-(3,4,5-trimethoxyphenyl)-2-propen-1-one (Compound 39)

Substantially the same procedure as in Example 1 was repeated using 3',4',5'-trimethoxyacetophenone (659 mg) and 6-methoxyindole-3-carboxaldehyde (550 mg) except that the reaction solution was concentrated under reduced pressure, that the residue was purified by silica gel column chromatography, and that the obtained crude crystals were recrystallized from ethanol, to give Compound 39 (369 mg).

$^1$H-NMR (270 MHz, $CDCl_3$) δ 3.88 (s, 3H), 3.94 (s, 3H), 3.97 (s, 6H), 6.96 (m, 2H), 7.30 (s, 2H), 7.48 (d, J = 15.4 Hz, 1H), 7.53 (s, 1H), 7.86 (d, J = 8.9 Hz, 1H), 8.05 (d, J = 15.4 Hz, 1H), 8.52 (brs, 1H)
EI-MS m/z = 367 ($M^+$)

Example 40

(E)-3-(6,7-Dimethoxyindol-3-yl)-1-(3,4,5-trimethoxyphenyl)-2-propen-1-one (Compound 40)

Process 1

Substantially the same procedure as in Process 1 of Example 15 was repeated using 6,7-dimethoxyindole (590 mg) to give 6,7-dimethoxyindole-3-carboxaldehyde (577 mg).

$^1$H-NMR (90 MHz, $CDCl_3$) δ 3.94 (s, 3H), 4.02 (s, 3H), 7.00 (d, J = 8.5 Hz, 1H), 7.74 (d, J = 3.1 Hz, 1H), 7.93 (d, J = 8.5 Hz, 1H), 8.95 (brs, 1H), 10.00 (s, 1H)
EI-MS m/z = 205 ($M^+$)

Process 2

Substantially the same procedure as in Example 1 was repeated using 3',4',5'-trimethoxyacetophenone (525 mg) and 6,7-dimethoxyindole-3-carboxaldehyde obtained in the above Process 1 except that water was added to the reaction solution, that the precipitated crystals were collected by filtration, and that the obtained crude crystals were recrystallized from ethanol, to give Compound 40 (586 mg).

$^1$H-NMR (270 MHz, $CDCl_3$) δ 3.94 (s, 3H), 3.95 (s, 3H), 3.97 (s, 6H), 4.04 (s, 3H), 7.02 (d, J = 8.7 Hz, 1H), 7.30 (s, 2H), 7.46 (d, J = 15.6 Hz, 1H), 7.55 (d, J = 3.0 Hz, 1H), 7.62 (d, J = 8.7 Hz, 1H), 8.04 (d, J = 15.6 Hz, 1H), 8.71 (s, 1H)
EI-MS m/z = 397 ($M^+$)

Example 41

(E)-3-(6-Hydroxy-7-methoxyindol-3-yl)-1-(3,4,5-trimethoxyphenyl)-2-propen-1-one (Compound 41)

Process 1

Substantially the same procedure as in Process 1 of Example 15 was repeated using 6-benzyloxy-7-methoxyindole (3.2 g) to give 6-benzyloxy-7-methoxyindole-3-carboxaldehyde (3.42 g).

$^1$H-NMR (90 MHz, $CDCl_3$) δ 4.04 (s, 3H), 5.17 (s, 2H), 7.00 (d, J = 8.5 Hz, 1H), 7.39 (m, 5H), 7.75 (d, J = 2.8 Hz, 1H), 7.89 (d, J = 8.5 Hz, 1H), 8.85 (brs, 1H), 10.00 (s, 1H)
EI-MS m/z = 281 ($M^+$)

Process 2

Substantially the same procedure as in Process 2 of Example 27 was repeated using 6-benzyloxy-7-methoxyindole-3-carboxaldehyde (1.41 g) obtained in the above Process 1 to give 6-hydroxy-7-methoxyindole-3-carboxaldehyde (0.47 g).

$^1$H-NMR (90 MHz, DMSO-$d_6$) δ 3.88 (s, 3H), 6.79 (d, J = 8.4 Hz, 1H), 7.58 (d, J = 8.4 Hz, 1H), 7.99 (s, 1H), 9.06 (s, 1H), 9.84 (s, 1H), 11.94 (s, 1H)
EI-MS m/z = 191 ($M^+$)

Process 3

Substantially the same procedure as in Example 1 was repeated using 3',4',5'-trimethoxyacetophenone (420 mg) and 6-hydroxy-7-methoxyindole-3-carboxaldehyde (382 mg) obtained in the above Process 2 except that the reaction solution was concentrated under reduced pressure, that the residue was purified by silica gel column chromatography, and that the obtained crude crystals were recrystallized from ethyl acetate and then from a mixed solvent of ethanol and water, to give Compound 41 (214 mg).

$^1$H-NMR (270 MHz, $CDCl_3$) δ 3.92 (s, 3H), 3.96 (s, 6H), 4.01 (s, 3H), 6.94 (d, J = 8.4 Hz, 1H), 7.40 (s, 2H), 7 41 (d, J = 15.5 Hz, 1H), 7.50 (d, J = 8.4 Hz, 1H), 7.53 (d, J = 3.0 Hz, 1H), 8.03 (d, J = 15.5 Hz, 1H), 8.14 (s, 1H), 10.72 (s, 1H)
EI-MS m/z = 383 ($M^+$)

Example 42

(E)-3-(6-Chloroindol-3-yl)-1-(3,4,5-trimethoxyphenyl)-2-propen-1-one (Compound 42)

Substantially the same procedure as in Example 1 was repeated using 3',4',5'-trimethoxyacetophenone (2.1 g) and 6-chloroindole-3-carboxaldehyde (1.8 g) except that the obtained product was recrystallized from ethyl acetate, to give Compound 42 (2.57 g).

$^1$H-NMR (270 MHz, DMSO-$d_6$) δ 3.77 (s, 3H), 3.91 (s, 6H), 7.21 (dd, J = 8.7, 1.8 Hz, 1H), 7.37 (s, 2H), 7.53 (d, J = 1.8 Hz, 1H), 7.62 (d, J = 15.8 Hz, 1H), 8.01 (d, J = 15.8 Hz, 1H), 8.07 (d, J = 8.7 Hz, 1H), 8.17 (s, 1H), 11.98 (s, 1H)
EI-MS m/z = 371, 373 ($M^+$)

Example 43

(E)-3-(7-Methylindol-3-yl)-1-(3,4,5-trimethoxyphenyl)-2-propen-1-one (Compound 43)

Process 1

Substantially the same procedure as in Process 1 of Example 15 was repeated using 7-methylindole (500 mg) to give 7-methylindole-3-carboxaldehyde (595 mg).

$^1$H-NMR (90 MHz, $CDCl_3$) δ 2.52 (s, 3H), 7.15-7.30 (m, 2H), 7.89 (s, 1H), 8.15 (dd, J = 7.5, 1.5 Hz, 1H), 8.80 (brs,

1H), 10.06 (s, 1H)
EI-MS m/z = 159 ($M^+$)

Process 2

Substantially the same procedure as in Example 12 was repeated using 3',4',5'-trimethoxyacetophenone (630 mg) and 7-methylindole-3-carboxaldehyde (480 mg) obtained in the above Process 1 to give Compound 43 (610 mg) .

$^1$H-NMR (270 MHz, $CDCl_3$) δ 2.54 (s, 3H), 3.94 (s, 3H), 3.97 (s, 6H), 7.13 (d, J = 7.4 Hz, 1H), 7.22 (t, J = 7.4 Hz, 1H), 7.31 (s, 2H), 7.53 (d, J = 15.8 Hz, 1H), 7.64 (d, J = 2.5 Hz, 1H), 7.85 (d, J = 7.4 Hz, 1H), 8.10 (d, J = 15.8 Hz, 1H), 8.58 (brs, 1H)
EI-MS m/z = 351 ($M^+$)

Example 44

(E)-3-(7-Nitroindol-3-yl)-1-(3,4,5-trimethoxyphenyl)-2-propen-1-one (Compound 44)

Substantially the same procedure as in Example 1 was repeated using 3',4',5'-trimethoxyacetophenone (630 mg) and 7-nitroindole-3-carboxaldehyde (552 mg) to give Compound 44 (491 mg).

$^1$H-NMR (270 MHz, $CDCl_3$) δ 3.94 (s, 3H), 3.98 (s, 6H), 7.37 (s, 1H), 7.38 (t, J = 7.9 Hz, 1H), 7.53 (d, J = 15.8 Hz, 1H), 7.90 (d, J = 3.0 Hz, 1H), 8.10 (d, J = 15.8 Hz, 1H), 8.25 (d, J = 7.9 Hz, 1H), 8.10 (d, J = 7.9 Hz, 1H), 11.56 (brs, 1H)
EI-MS m/z = 382 ($M^+$)

Example 45

(E)-3-(7-Nitro-1-methylindol-3-yl)-1-(3,4,5-trimethoxyphenyl)-2-propen-1-one (Compound 45)

Substantially the same procedure as in Example 2 was repeated using Compound 44 (435 mg) obtained in Example 44 except that insoluble matters were filtered off, that the resulting filtrate was concentrated under reduced pressure, that water was added thereto, and that the precipitated crystals were collected by filtration, to give Compound 45 (417 mg)

$^1$H-NMR (270 MHz, $CDCl_3$) δ 3.91 (s, 3H), 3.95 (s, 3H), 3.97 (s, 6H), 7.29 (s, 2H), 7.33 (t, J = 7.9 Hz, 1H), 7.46 (d, J = 15.5 Hz, 1H), 7.59 (s, 1H), 7.89 (d, J = 7.9 Hz, 1H), 8.05 (d, J = 15.5 Hz, 1H), 8.20 (d, J = 7.9 Hz, 1H)
EI-MS m/z = 396 ($M^+$)

Example 46

(E)-3-(7-Methoxyindol-3-yl)-1-(3,4,5-trimethoxyphenyl)-2-propen-1-one (Compound 46)

Substantially the same procedure as in Example 39 was repeated using 3',4',5'-trimethoxyacetophenone (1.74 g) and 7-methoxyindole-3-carboxaldehyde (1.45 g) to give Compound 46 (1.47 g).

$^1$H-NMR (270 MHz, $CDCl_3$) δ 3.94 (s, 3H), 3.97 (s, 6H), 3.99 (s, 3H), 6.77 (d, J = 7.9 Hz, 1H), 7.23 (t, J = 7.9 Hz, 1H), 7.31 (s, 2H), 7.51 (d, J = 15.5 Hz, 1H), 7.58 (d, J = 7.9 Hz, 1H), 7.59 (d, J = 2.5 Hz, 1H), 8.09 (d, J = 15.5 Hz, 1H), 8.79 (brs, 1H)
EI-MS m/z = 367 ($M^+$)

Example 47

(E)-3-(1-Benzylindol-3-yl)-1-(3,4,5-trimethoxyphenyl)-2-propen-1-one (Compound 47)

Substantially the same procedure as in Example 3 was repeated using Compound 1 (2.37 g) obtained in Example 1 and benzyl bromide (1.8 g) except that the obtained crude crystals were recrystallized from ethanol and then from a mixed solvent of ethyl acetate and hexane, to give Compound 47 (2.11 g).

$^1H$-NMR (270 MHz, $CDCl_3$) δ 3.94 (s, 3H), 3.96 (s, 6H), 5.35 (s, 2H), 7.15 (m, 2H), 7.22-7.39 (m, 8H), 7.49 (d, J = 15.5 Hz, 1H), 7.55 (s, 1H), 7.99 (m, 1H), 8.08 (d, J = 15.5 Hz, 1H)
EI-MS m/z = 427 ($M^+$)

Example 48

(E)-3-(Indol-3-yl)-2-methyl-1-(3,4,5-trimethoxyphenyl)-2-propen-1-one (Compound 48)

Process 1

3,4,5-Trimethoxybenzaldehyde (3.92 g) was dissolved in tetrahydrofuran (100 ml), and ethylmagnesium bromide (1M tetrahydrofuran solution, 22 ml) was added thereto under ice-cooling. A 10 % aqueous solution of citric acid was added to the reaction solution, and the mixture was extracted with ethyl acetate. The organic layer was washed with water and then with a saturated saline, dried over magnesium sulfate, and concentrated under reduced pressure. The residue was dissolved in acetone (100 ml), and Jones' reagent was added thereto under ice-cooling, followed by stirring for 30 minutes. 2-Propanol (10 ml) was added to the reaction solution, and the solution was then concentrated under reduced pressure, followed by partitioning between ethyl acetate and water. The organic layer was washed with water and then a saturated saline, dried over magnesium sulfate, and concentrated under reduced pressure. The residue was purified by silica gel column chromatography to give 3,'4',5'-trimethoxypropiophenone (2.3 g).

$^1H$-NMR (90 MHz, $CDCl_3$) δ 1.23 (t, J = 7.2 Hz, 3H), 2.97 (q, J = 7.2 Hz, 2H), 3.92 (s, 9H), 7.22 (s, 2H)
EI-MS m/z = 224 ($M^+$)

Process 2

Substantially the same procedure as in Example 1 was repeated using 3',4',5'-trimethoxypropiophenone (1.12 g) obtained in the above Process 1 and indole-3-carboxaldehyde (0.73 g) except that the obtained crystals were recrystallized from a mixed solvent of ethanol, acetone, ethyl acetate, N,N-dimethylformamide and water, to give Compound 48 (0.81 g).

$^1H$-NMR (270 MHz, DMSO-$d_6$) δ 2.23 (s, 3H), 3.79 (s, 3H), 3.82 (s, 6H), 6.96 (s, 2H), 7.11 (m, 1H), 7.19 (m, 1H), 7.47 (m, 2H), 7.64 (s, 1H), 7.89 (s, 1H), 11.90 (s, 1H)
EI-MS m/z = 351 ($M^+$)

Example 49

(E)-3-(Indol-3-yl)-2-propyl-1-(3,4,5-trimethoxyphenyl)-2-propen-1-one (Compound 49)

Process 1

Substantially the same procedure as in Process 1 of Example 48 was repeated using 3,4,5-trimethoxybenzaldehyde (3.92 g) and butyl lithium (1.56 M hexane solution, 15 ml) to give 1-(3,4,5-trimethoxyphenyl)-1-pentanone (2.84 g).

$^1H$-NMR (90 MHz, $CDCl_3$) δ 0.96 (t, J = 6.3 Hz, 3H), 1.20-1.90 (m, 4H), 2.93 (t, J = 7.0 Hz, 2H), 3.92 (s, 9H), 7.20 (s, 2H)
EI-MS m/z = 252 ($M^+$)

Process 2

Substantially the same procedure as in Example 1 was repeated using 1-(3,4,5-trimethoxyphenyl)-1-pentanone (1.26 g) obtained in the above Process 1 and indole-3-carboxaldehyde (0.73 g) except that water was added to the reaction solution, that the precipitated crystals were collected by filtration, and that the obtained crude crystals were recrystallized from a mixed solvent of ethanol and water, to give Compound 49 (0.51 g).

$^1H$-NMR (270 MHz, DMSO-$d_6$) δ 1.04 (t, J = 7.6 Hz, 3H), 1.60 (m, 2H), 2.71 (t, J = 7.6 Hz, 2H), 3.79 (s, 3H), 3.81 (s, 6H), 6.96 (s, 2H), 7.09 (m, 1H), 7.18 (m, 1H), 7.42 (d, J = 6.4 Hz, 1H), 7.48 (d, J = 7.9 Hz, 1H), 7.53 (s, 1H), 7.77 (d, J = 2.5 Hz, 1H), 11.81 (s, 1H)

EI-MS m/z = 379 ($M^+$)

Example 50

(E)-3-[1-(4-Methylbenzoyl)indol-3-yl]-1-(3,4,5-trimethoxyphenyl)-2-propen-1-one (Compound 50)

Process 1

4-Methylbenzoic acid (2.72 g) and triethylamine (2.53 g) were suspended in dichloromethane (50 ml), and a dichloromethane solution (10 ml) of thionyl chloride (1.19 g) was added thereto, followed by stirring for one hour. The reaction solution was diluted with dichloromethane, and washed with a 10 % aqueous solution of citric acid. The organic layer was dried over anhydrous magnesium sulfate, and concentrated under reduced pressure to give acid anhydride (2.50 g) .

IR (KBr tablet) 1780, 1720 $cm^{-1}$
EI-MS m/z = 254 ($M^+$)

Process 2

Substantially the same procedure as in Example 3 was repeated using Compound 1 (1.35 g) obtained in Example 1 and the acid anhydride (2.03 g) obtained in the above Process 1 except that the reaction product was purified by silica gel column chromatography, and that the obtained crude crystals were recrystallized from a mixed solvent of ethanol and acetone, to give Compound 50 (1.48 g).

$^1$H-NMR (270 MHz, $CDCl_3$) $\delta$ 2.50 (s, 3H), 3.95 (s, 3H), 3.96 (s, 6H), 7.28 (s, 2H), 7.38 (d, J = 7.9 Hz, 2H), 7.47 (m, 2H), 7.59 (d, J = 15.8 Hz, 1H), 7.68 (d, J = 7.9 Hz, 2H), 7.73 (s, 1H), 7.92 (d, J = 15.8 Hz, 1H), 7.92 (m, 1H), 8.41 (m, 1H)
EI-MS m/z = 455 ($M^+$)

Example 51

(E)--3-[1-(4-Methoxybenzoyl)indol-3-yl]-1-(3,4,5-trimethoxyphenyl)-2-propen-1-one (Compound 51)

Process 1

Substantially the same procedure as in Process 1 of Example 50 was repeated using 4-methoxybenzoic acid (3.04 g) to give an acid anhydride (2.87 g).

IR (KBr tablet) 1780, 1720 $cm^{-1}$
EI-MS m/z = 286 ($M^+$)

Process 2

Substantially the same procedure as in Example 3 was repeated using Compound 1 (1.35 g) obtained in Example 1 and the acid anhydride (2.29 g) obtained in the above Process 1 except that the reaction product was purified by silica gel column chromatography, and that the obtained crude crystals were recrystallized from ethanol, to give Compound 51 (1.08 g) .

$^1$H-NMR (270 MHz, $CDCl_3$) $\delta$ 3.93 (s, 3H), 3.95 (s, 3H), 3.96 (s, 6H), 7.05 (d, J = 8.7 Hz, 2H), 7.29 (s, 2H), 7.45 (m, 2H), 7.70 (d, J = 15.6 Hz, 1H), 7.76 (s, 1H), 7.78 (d, J = 8.7 Hz, 2H), 7.94 (d, J = 15.6 Hz, 1H), 7.97 (m, 1H), 8.35 (m, 1H)
EI-MS m/z = 471 ($M^+$)

Example 52

(E)-3-[1-(3-Methylbenzoyl)indol-3-yl]-1-(3,4,5-trimethoxyphenyl)-2-propen-1-one (Compound 52)

Process 1

Substantially the same procedure as in Process 1 of Example 50 was repeated using 3-methylbenzoic acid (2.72 g) to give an acid anhydride (2.58 g).

IR (KBr tablet) 1780, 1720 $cm^{-1}$
EI-MS m/z = 254 ($M^+$)

Process 2

Substantially the same procedure as in Process 2 of Example 51 was repeated using Compound 1 (1.35 g) obtained in Example 1 and the acid anhydride (2.03 g) obtained in the above Process 1 to give Compound 52 (1.65 g).

$^1$H-NMR (270 MHz, $CDCl_3$) δ 2.48 (s, 3H), 3.95 (s, 3H), 3.96 (s, 6H), 7.28 (s, 2H), 7.47 (m, 4H), 7.53 (m, 1H), 7.58 (brs, 1H), 7.59 (d, J = 15.6 Hz, 1H), 7.70 (s, 1H), 7.91 (d, J = 15.6 Hz, 1H), 7.97 (m, 1H), 8.43 (m, 1H)
EI-MS m/z = 455 ($M^+$)

Example 53

(E)-3-[1-(3-Methoxybenzoyl)indol-3-yl]-1-(3,4,5-trimethoxyphenyl)-2-propen-1-one (Compound 53)

Process 1

Substantially the same procedure as in Process 1 of Example 50 was repeated using 3-methoxybenzoic acid (3.04 g) to give an acid anhydride (2.93 g).

IR (KBr tablet) 1780, 1720 $cm^{-1}$
EI-MS m/z = 286 ($M^+$)

Process 2

Substantially the same procedure as in Process 2 of Example 50 was repeated using Compound 1 (1.35 g) obtained in Example 1 and the acid anhydride (2.29 g) obtained in the above Process 1 to give Compound 53 (1.46 g).

$^1$H-NMR (270 MHz, $CDCl_3$) δ 3.89 (s, 3H), 3.95 (s, 3H), 3.96 (s, 6H), 7.19 (m, 1H), 7.28 (s, 2H), 7.29 (m, 2H), 7.48 (m, 3H), 7.59 (d, J = 15.6 Hz, 1H), 7.72 (s, 1H), 7.91 (d, J = 15.6 Hz, 1H), 7.97 (m, 1H), 8.43 (m, 1H)
EI-MS m/z = 471 ($M^+$)

Example 54

(E)-3-[1-(2-Methylbenzoyl)indol-3-yl]-1-(3,4,5-trimethoxyphenyl)-2-propen-1-one (Compound 54)

Process 1

Substantially the same procedure as in Process 1 of Example 50 was repeated using 2-methylbenzoic acid (2.72 g) to give an acid anhydride (2.55 g).

IR (KBr tablet) 1780, 1720 $cm^{-1}$
EI-MS m/z = 254 ($M^+$)

Process 2

Substantially the same procedure as in Process 2 of Example 50 was repeated using Compound 1 (1.35 g) obtained in Example 1 and the acid anhydride (2.03 g) obtained in the above Process 1 to give Compound 54 (1.45 g).

$^1$H-NMR (270 MHz, $CDCl_3$) δ 2.37 (s, 3H), 3.94 (s, 3H), 3.95 (s, 6H), 7.27 (s, 2H), 7.33-7.51 (m, 7H), 7.56 (d, J = 15.8 Hz, 1H), 7.85 (d, J = 15.8 Hz, 1H), 7.95 (m, 1H), 8.41 (m, 1H)
EI-MS m/z = 455 ($M^+$)

Example 55

(E)-3-(1-(2-Methoxybenzoyl)indol-3-yl]-1-(3,4,5-trimethoxyphenyl)-2-propen-1-one (Compound 55)

Process 1

Substantially the same procedure as in Process 1 of Example 50 was repeated using 2-methoxybenzoic acid (3.04 g) to give an acid anhydride (2.90 g).

IR (KBr tablet) 1780, 1720 $cm^{-1}$
EI-MS m/z = 286 ($M^+$)

Process 2

Substantially the same procedure as in Process 2 of Example 50 was repeated using Compound 1 (1.35 g) obtained in Example 1 and the acid anhydride (2.29 g) obtained in the above Process 1 to give Compound 55 (0.98 g).

$^1$H-NMR (270 MHz, $CDCl_3$) δ 3.80 (s, 3H), 3.94 (s, 3H) , 3.95 (s, 6H), 7.07 (d, J = 8.4 Hz, 1H), 7.13 (td, J = 7.5, 1.0 Hz, 1H), 7.27 (s, 2H), 7.45 (s, 1H), 7.48 (m, 3H), 7.56 (d, J = 15.8 Hz, 1H), 7.57 (m, 1H), 7.86 (d, J = 15.8 Hz, 1H), 7.94 (m, 1H), 8.50 m, 1H)
EI-MS m/z = 471 ($M^+$)

Example 56

(E)-3-[1-(4-Acetamidobenzoyl)indol-3-yl]-1-(3,4,5-trimethoxyphenyl)-2-propen-1-one (Compound 56)

Process 1

4-Acetamidobenzoic acid (2.15 g) was dissolved in a mixed solvent of dichloromethane (10 ml) and tetrahydrofuran (10 ml), and thionyl chloride (1.43 g) and pyridine (20 mℓ) were added thereto, followed by heating under reflux for one hour. After the reaction solution was cooled, the solution was poured into a tetrahydrofuran solution (5 ml) of p-nitrophenol (1.39 g), which was separately prepared, under ice-cooling. Triethylamine (2.43 g) was further added to the mixture, followed by stirring for 14 hours. The reaction solution was diluted with chloroform, and washed with a 10 % aqueous solution of citric acid. The crystals precipitated in the organic layer were collected by filtration to give an activated ester (2.41 g).

$^1$H-NMR (90 MHz, DMSO-$d_6$) δ 2.12 (s, 3H), 7.57 (d, J = *9.1 Hz, 2H*), *7.79* (*d*, *J = 8.9 Hz, 2H*), *8.09* (*d*, *J* = 8.9 Hz, 2H), 8.32 (d, J = 9.1 Hz, 2H), 10.33 (s, 1H)
EI-MS m/z = 300 ($M^+$)

Process 2

Substantially the same procedure as in Process 2 of Example 50 was repeated using Compound 1 (1.35 g) obtained in Example 1 and the activated ester (2.40 g) obtained in the above Process 1 to give Compound 56 (1.37 g).

$^1$H-NMR (270 MHz, $CDCl_3$) δ 2.26 (s, 3H), 3.95 (s, 3H), 3.96 (s, 6H), 7.29 (s, 2H), 7.47 (m, 2H), 7.56 (s, 1H), 7.59 (d, J = 15.8 Hz, 1H), 7.732 (d, J = 9.2 Hz, 2H), 7.733 (s, 1H), 7.78 (d, J = 9.2 Hz, 2H), 7.93 (d, J = 15.8 Hz, 1H), 7.97 (m, 1H), 8.37 (m, 1H)
FAB-MS m/z = 499 ($M^+$ + 1)

Example 57

(E)-3-[1-(4-Dimethylaminobenzoyl)indol-3-yl]-1-(3,4,5-trimethoxyphenyl)-2-propen-1-one (Compound 57)

Process 1

Substantially the same procedure as in Process 1 of Example 56 was repeated using 4-dimethylaminobenzoic acid (2.15 g) except that the crystals were not precipitated to give a chloroform solution of an activated ester.

Process 2

Substantially the same procedure as in Process 2 of Example 50 was repeated using Compound 1 (3.38 g) obtained in Example 1 and the chloroform solution of an activated ester obtained in the above Process 1, to give Compound 57 (2.95 g).

$^1$H-NMR (270 MHz, $CDCl_3$) δ 3.12 (s, 6H), 3.95 (s, 3H), 3.97 (s, 6H), 6.75 (d, J = 8.9 Hz, 2H), 7.30 (s, 2H), 7.43 (m, 2H), 7.59 (d, J = 15.6 Hz, 1H), 7.74 (d, J = 8.9 Hz, 2H), 7.86 (s, 1H), 7.97 (m, 1H), 7.98 (d, J = 15.6 Hz, 1H), 8.29 (m, 1H)
EI-MS m/z = 484 ($M^+$)

Example 58

(E)-3-[1-(3,4-Dimethoxybenzoyl)indol-3-yl]-1-(3,4,5-trimethoxyphenyl)-2-propen-1-one (Compound 58)

Process 1

Substantially the same procedure as in Process 1 of Example 56 was repeated using 3,4-dimethoxybenzoic acid (1.39 g) except that the crystals were not precipitated and that the organic layer was concentrated under reduced pressure, to give an activated ester (3.30 g).

$^1$H-NMR (90 MHz, $CDCl_3$) δ 3.97 (s, 3H), 3.98 (s, 3H), 6.97 (d, J = 8.5 Hz, 1H), 7.40 (d, J = 9.0 Hz, 2H), 7.65 (d, J = 1.9 Hz, 1H), 7.87 (dd, J = 8.5, 1.9 Hz, 1H), 8.32 (d, J = 9.0 Hz, 2H)
EI-MS m/z = 303 ($M^+$)

Process 2

Substantially the same procedure as in Process 2 of Example 50 was repeated using Compound 1 (1.69 g) obtained in Example 1 and the activated ester obtained in the above Process 1 to give Compound 58 (0.53 g).

$^1$H-NMR (270 MHz, $CDCl_3$) δ 3.95 (s, 3H), 3.97 (s, 9H), 4.01 (s, 3H), 7.00 (d, J = 8.9 Hz, 1H), 7.29 (s, 2H), 7.38 (m, 2H), 7.47 (m, 2H), 7.61 (d, J = 15.5 Hz, 1H), 7.81 (s, 1H), 7.95 (d, J = 15.5 Hz, 1H), 7.97 (m, 1H), 8.35 (m, 1H)
EI-MS m/z = 501 ($M^+$)

Example 59

(E)-3-[1-(3,4,5-Trimethoxybenzoyl)indol-3-yl]-1-(3,4,5-trimethoxyphenyl)-2-propen-1-one (Compound 59)

Substantially the same procedure as in Example 3 was repeated using Compound 1 (1.69 g) obtained in Example 1 and 3,4,5-trimethoxybenzoyl chloride (2.31 g) except that the obtained crude crystals were recrystallized from a mixed solvent of ethanol and acetone, to give Compound 59 (1.22 g) .

$^1$H-NMR (270 MHz, $CDCl_3$) δ 3.91 (s, 6H), 3.95 (s, 3H), 3.96 (s, 6H), 3.97 (s, 3H), 7.00 (s, 2H), 7.29 (s, 2H), 7.48 (m, 2H), 7.61 (d, J = 15.8 Hz, 1H), 7.78 (s, 1H), 7.94 (d, J = 15.8 Hz, 1H), 7.99 (m, 1H), 8.37 (m, 1H)
EI-MS m/z = 531 ($M^+$)

Example 60

(E)-3-(1-Nicotinoylindol-3-yl)-1-(3,4,5-trimethoxyphenyl)-2-propen-1-one (Compound 60)

Substantially the same procedure as in Example 59 was repeated using Compound 1 (1.43 g) obtained in Example 1 and nicotinoyl chloride hydrochloride (1.51 g) to give Compound 60 (0.37 g).

$^1$H-NMR (270 MHz, $CDCl_3$) $\delta$ 3.95 (s, 3H), 3.96 (s, 6H), 7.28 (s, 2H), 7.47-7.66 (m, 5H), 7.90 (d, J = 15.8 Hz, 1H), 7.98 (m, 1H), 8.14 (brd, J = 7.9 Hz, 1H), 8.46 (m, 1H), 8.91 (brd, J = 4.6 Hz, 1H), 9.03 (d, J = 2.0 Hz, 1H)
FAB-MS m/z = 443 ($M^+$ + 1)

Example 61

(E)-3-(1-Isonicotinoylindol-3-yl)-1-(3,4,5-trimethoxyphenyl)-2-propen-1-one (Compound 61)

Substantially the same procedure as in Example 59 was repeated using Compound 1 (1.43 g) obtained in Example 1 and isonicotinoyl chloride hydrochloride (1.51 g) to give Compound 61 (0.48 g).

$^1$H-NMR (270 MHz, $CDCl_3$) $\delta$ 3.95 (s, 3H), 3.96 (s, 6H), 7.28 (s, 2H), 7.47-7.65 (m, 6H), 7.89 (d, J = 15.5 Hz, 1H), 7.98 (m, 1H), 8.46 (m, 1H), 8.91 (m, 2H)
FAB-MS m/z = 443 ($M^+$ + 1)

Example 62

(E)-2-Ethyl-3-(indol-3-yl)-1-(3,4,5-trimethoxyphenyl)-2-propen-1-one (Compound 62)

Process 1

Substantially the same procedure as in Process 1 of Example 48 was repeated using 3,4,5-trimethoxybenzaldehyde (43.12 g) and propylmagnesium bromide (1.0 M tetrahydrofuran solution, 330 ml) to give 1-(3,4,5-trimethoxyphenyl)-1-butanone (17.43 g).

$^1$H-NMR (90 MHz, $CDCl_3$) $\delta$ 1.01 (t, J = 7.2 Hz, 3H), 1.77 (m, 2H), 2.91 (t, J = 7.2 Hz, 2H), 3.92 (s, 9H), 7.22 (s, 2H)
EI-MS m/z = 238 ($M^+$)

Process 2

Substantially the same procedure as in Example 1 was repeated using 1-(3,4,5-trimethoxyphenyl)-1-butanone (1.90 g) obtained in the above Process 1 and indole-3-carboxaldehyde (1.16 g) except that the reaction solution was concentrated under reduced pressure, that the residue was purified by silica gel column chromatography, and that the obtained crude crystals were recrystallized from ethanol, to give Compound 62 (0.88 g).

$^1$H-NMR (270 MHz, $CDCl_3$) $\delta$ 1.27 (t, J = 7.4 Hz, 3H), 2.83 (q, J = 7.4 Hz, 2H), 3.90 (s, 6H), 3.95 (s, 3H), 7.03 (s, 2H), 7.22 (m, 1H), 7.28 (m, 1H), 7.44 (d, J = 8.4 Hz, 1H), 7.54 (d, J = 7.4 Hz, 1H), 7.57 (s, 1H), 7.63 (d, J = 3.0 Hz, 1H), 8.62 (s, 1H)
EI-MS m/z = 365 ($M^+$)

Example 63

(E)-2-Methyl-3-(6-methylindol-3-yl)-1-(3,4,5-trimethoxyphenyl)-2-propen-1-one (Compound 63)

Substantially the same procedure as in Example 1 was repeated using 3,4,5-trimethoxypropiophenone (0.67 g) obtained in Process 1 of Example 48 and 6-methylindole-3-carboxaldehyde (0.48 g) obtained in Process 1 of Example 32 except that the reaction solution was concentrated under reduced pressure, that the residue was purified by silica gel column chromatography, and that the obtained crude crystals were recrystallized from a mixed solvent of hexane and ethyl acetate, and then from ethanol, to give Compound 62 (0.17 g).

$^1$H-NMR (270 MHz, $CDCl_3$) $\delta$ 2.30 (d, J = 1.0 Hz, 3H) , 2.47 (s, 3H), 3.89 (s, 6H), 3.95 (s, 3H), 7.021 (s, 2H), 7.024

(brd, J = 8.2 Hz, 1H), 7.22 (s, 1H), 7.45 (d, J = 8.2 Hz, 1H), 7.56 (d, J = 2.5 Hz, 1H), 7.66 (s, 1H), 8.53 (s, 1H)
EI-MS m/z = 365 ($M^+$)

Example 64

3-(Indol-3-yl)-2-phenyl-1-(3,4,5-trimethoxyphenyl)-2-propen-1-one (Compound 64)

Process 1

Substantially the same procedure as in Process 1 of Example 48 was repeated using 3,4,5-trimethoxybenzaldehyde (3.92 g) and benzylmagnesium bromide (2.0 M diethyl ether solution, 23 ml) to give 3,4,5-trimethoxydeoxybenzoin (3.51 g) .

$^1$H-NMR (90 MHz, $CDCl_3$) δ 3.84 (s, 6H), 3.89 (s, 3H), 4.22 (s, 2H), 7.25 (s, 7H)
EI-MS m/z = 286 ($M^+$)

Process 2

Substantially the same procedure as in Example 1 was repeated using 3,4,5-trimethoxydeoxybenzoin (1.43 g) obtained in the above Process 1 and indole-3-carboxyaldehdye (0.73 g) except that the reaction solution was concentrated under reduced pressure, that the residue was purified by silica gel column chromatography, and that the obtained crude product was further purified by preparative medium-pressure silica gel column chromatography, to give Compound 64 (0.89 g).
EI-MS m/z = 413 ($M^+$)

Example 65

(E)-2-Butyl-3-(indol-3-yl)-1-(3,4,5-trimethoxyphenyl)-2-propen-1-one (Compound 65)

Process 1

Substantially the same procedure as in Process 1 of Example 48 was repeated using 3,4,5-trimethoxybenzaldehyde (3.92 g) and pentylmagnesium bromide (2.0 M diethyl ether solution, 12 ml) to give 1-(3,4,5-trimethoxyphenyl)-1-hexanone (3.27 g).

$^1$H-NMR (90 MHz, $CDCl_3$) δ 0.92 (t, J = 5.9 Hz, 3H), 1.34 (m, 4H), 1.74 (m, 2H), 2.92 (t, J = 7.2 Hz, 2H), 3.91 (s, 9H), 7.22 (s, 2H)
EI-MS m/z = 266 ($M^+$)

Process 2

Substantially the same procedure as in Example 1 was repeated using 1-(3,4,5-trimethoxyphenyl)-1-hexanone (2.66 g) obtained in the above Process 1 and indole-3-carboxaldehyde (1.45 g) except that the reaction solution was concentrated under reduced pressure, that the residue was purified by silica gel column chromatography, and that the obtained crude crystals were recrystallized from ethyl acetate, to give Compound 65 (0.33 g).

$^1$H-NMR (270 MHz, $CDCl_3$) δ 0.97 (m, 3H), 1.53 (m, 2H), 1.63 (m, 2H), 2.81 (t, J = 7.7 Hz, 2H), 3.89 (s, 6H), 3.95 (s, 3H), 7.04 (s, 2H), 7.18 (m, 1H), 7.27 (m, 1H), 7.43 (d, J = 8.4 Hz, 1H), 7.54 (d, J = 8.4 Hz, 1H), 7.56 (s, 1H), 7.59 (d, J = 2.9 Hz, 1H), 8.69 (s, 1H)
EI-MS m/z = 393 ($M^+$)

Example 66

(E)-3-(6-Ethylindol-3-yl)-2-methyl-1-(3,4,5-trimethoxyphenyl)-2-propen-1-one (Compound 66)

Process 1

6-Hydroxymethyl-1-toluenesulfonylindole (2.50 g) was dissolved in ethyl acetate (240 ml), and manganese

dioxide (24 g) was added thereto, followed by stirring at room temperature for 24 hours. Manganese dioxide was filtered off and the filtrate was concentrated under reduced pressure. The residue was dissolved in tetrahydrofuran (100 ml), the resultant solution was cooled to -78°C, and a pentane solution of methyllithium (1M solution, 10 ml) was added thereto. One hour after, acetic anhydride (1 ml) was added to the solution at the same temperature, followed by partitioning between ethyl acetate and water. The organic layer was dried over anhydrous magnesium sulfate and concentrated under reduced pressure. The residue was dissolved in acetic acid (10 ml), and 10 % palladium on carbon (1.63 g) was added thereto, followed by stirring at room temperature for 48 hours under an atmosphere of hydrogen. The catalyst was filtered off, the filtrate was concentrated under reduced pressure, and the residue was purified by silica gel column chromatography to give Compound (XIV) (0.78 g).

$^1$H-NMR (90 MHz, $CDCl_3$) δ 1.24 (t, J = 7.5 Hz, 3H), 2.36 (s, 3H), 2.65 (t, J = 7.5 Hz, 2H), 2.82 (dd, J = 8.7, 8.0 Hz, 2H), 3.90 (dd, J = 8.7, 8.0 Hz, 2H), 6.78 (d, J = 7.8 Hz, 1H), 6.97 (d, J = 7.8 Hz, 1H), 7.20 (d, J = 8.4 Hz, 2H), 7.49 (s, 1H), 7.67 (d, J = 8.4 Hz, 2H)
EI-MS m/z = 301 ($M^+$)

Process 2

Compound (XIV) (1.12 g) obtained in the above Process 1 was dissolved in chlorobenzene (50 ml), and manganese dioxide (7.72 g) was added thereto, followed by stirring at room temperature for 72 hours. Manganese dioxide was filtered off, and the filtrate was concentrated under reduced pressure. The residue was dissolved in methanol (30 ml), and potassium hydroxide (1.16 g) was added thereto, followed by heating under reflux for 15 hours. The mixture was subjected to partitioning between chloroform and a 10 % aqueous solution of citric acid, and the organic layer was concentrated under reduced pressure. Substantially the same procedure as in Process 1 of Example 15 was repeated using the residue to give 6-ethylindole-3-carboxaldehyde (0.22 g).

$^1$H-NMR (90 MHz, $CDCl_3$) δ 1.27 (t, J = 7.6 Hz, 3H), 2.76 (q, J = 7.6 Hz, 2H), 7.11-7.24 (m, 2H), 7.76 (s, 1H), 8.20 (d, J = 8.2 Hz, 1H), 8.99 (brs, 1H), 10.01 (s, 1H)
EI-MS m/z = 173 ($M^+$)

Process 3

Substantially the same procedure as in Example 20 was repeated using 3',4',5'-trimethoxypropiophenone (0.42 g) obtained in Process 1 of Example 48 and 6-ethylindole-3-carboxaldehyde (0.37 g) obtained in the above Process 2 to give Compound 66 (0.19 g).

$^1$H-NMR (270 MHz, $CDCl_3$) δ 1.29 (t, J = 7.5 Hz, 3H), 2.31 (s, 3H), 2.77 (q, J = 7.5 Hz, 2H), 3.89 (s, 6H), 3.96 (s, 3H), 7.01 (d, J = 8.0 Hz, 1H), 7.02 (s, 2H), 7.25 (s, 1H), 7.48 (d, J = 8.0 Hz, 1H) , 7.58 (d, J = 2.5 Hz, 1H), 7.67 (s, 1H), 8.69 (brs, 1H)
EI-MS m/z = 379 ($M^+$)

<u>Example 67</u>

(E)-3-(1-Diglycolylindol-3-yl)-1-(3,4,5-trimethoxyphenyl)-2-propen-1-one (Compound 67)

Substantially the same procedure as in Example 3 was repeated using Compound 1 (0.17 g) obtained in Example 1 and diglycolic anhydride (0.12 g) except that the obtained crude crystals were washed with ethyl acetate, to give Compound 67 (0.48 g).

$^1$H-NMR (270 MHz, DMSO-$d_6$) δ 3.79 (s, 3H), 3.92 (s, 6H), 4.28 (s, 2H), 4.96 (s, 2H), 7.40 (s, 2H), 7.45 (m, 2H), 7.87 (d, J = 15.6 Hz, 1H), 7.95 (d, J = 15.8 Hz, 1H), 8.14 (m, 1H), 8.32 (m, 1H), 8.62 (s, 1H)
EI-MS m/z = 453 ($M^+$)

Example 68

(E)-3-(6-Isopropylindol-3-yl)-1-(3,4,5-trimethoxyphenyl)-2-propen-1-one (Compound 68)

Process 1

Substantially the same procedure as in Process 1 of Example 15 was repeated using 6-isopropylindole (3.18 g) except that the crystals precipitated in the reaction solution were filtered off, that the filtrate was extracted with ethyl acetate, and that the extract was dried over anhydrous magnesium sulfate and concentrated under reduced pressure, to give 6-isopropylindole-3-carboxaldehyde (0.39 g) .

$^1$H-NMR (90 MHz, $CDCl_3$) δ 1.31 (d, J = 8.7 Hz, 6H), 2.70 (m, 1H), 7.08 (m, 1H), 7.24 (s, 1H), 7.74 (brs, 1H), 8.18 (d, J = 9.0 Hz, 1H), 9.28 (s, 1H), 9.97 (s, 1H)
EI-MS m/z = 187 ($M^+$)

Process 2

Substantially the same procedure as in Process 2 of Example 62 was repeated using 3',4',5'-trimethoxypropiophenone (0.42 g) and 6-isopropylindole-3-carboxaldehyde (0.37 g) to give Compound 68 (0.19 g).

$^1$H-NMR (270 MHz, $CDCl_3$) δ 1.32 (d, J = 6.9 Hz, 6H), 3.05 (m, 1H), 3.95 (s, 3H), 3.96 (s, 6H), 7.22 (dd, J = 8.5, 1.5 Hz, 1H), 7.30 (s, 3H), 7.53 (d, J = 15.4 Hz, 1H), 7.58 (d, J = 2.5 Hz, 1H), 7.92 (d, J = 8.5 Hz, 1H), 8.08 (d, J = 15.4 Hz, 1H), 8.61 (s, 1H)
EI-MS m/z = 379 ($M^+$)

Industrial Applicability

According to the present invention, there can be provided propenone derivatives having an excellent antitumor activity.

**Claims**

**1.** A propenone derivative represented by the following formula (I) :

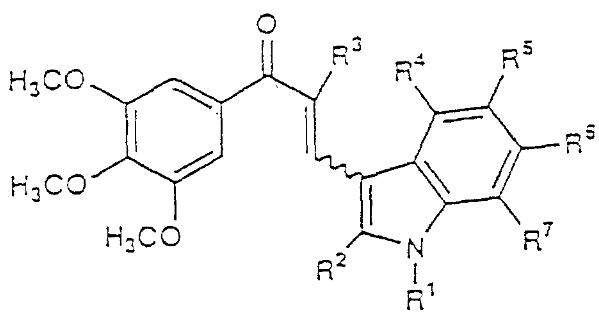

(I)

wherein $R^1$ represents hydrogen, $C_1$-$C_6$ alkyl, $C_1$-$C_7$ alkanoyl, $C_1$-$C_6$ alkoxycarbonyl, $C_1$-$C_6$ alkylsulfonyl, $C_7$-$C_{13}$ aralkyl,
substituted or unsubstituted aroyl, substituted or unsubstituted arylsulfonyl, substituted or unsubstituted heteroarylcarbonyl, substituted or unsubstituted heteroarylsulfonyl, diglycolyl or

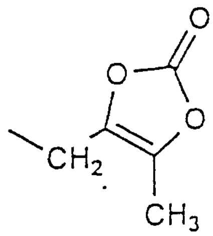 ;

$R^2$ represents hydrogen, $C_1$-$C_6$ alkyl, halogen, substituted or unsubstituted aryl, or substituted or unsubstituted heteroaryl; $R^3$ represents hydrogen, $C_1$-$C_6$ alkyl or substituted or unsubstituted aryl; and $R^4$, $R^5$, $R^6$ and $R^7$ represent independently hydrogen, $C_1$-$C_6$ alkyl, $C_1$-$C_6$ alkoxy, $C_7$-$C_{13}$ aralkyloxy, hydroxy, nitro, halogen, trifluoromethyl or -$NR^8R^9$ (wherein $R^8$ and $R^9$ represent independently hydrogen, $C_1$-$C_6$ alkyl, $C_1$-$C_7$ alkanoyl, $C_1$-$C_6$ alkoxycarbonyl or substituted or unsubstituted aroyl), or a pharmaceutically acceptable salt thereof,
wherein the above substituted groupe have 1 to 3 substituents selected from the group consisting of $C_1$-$C_6$ alkvl, $C_1$-$C_6$ alkoxy, $C_1$-$C_7$ alkanoyl, $C_1$-$C_6$ alkoxycarbonyl, hydroxy, amino, $C_1$-$C_6$ alkylamino, di($C_1$-$C_6$ alkyl)amino, $C_1$-$C_7$ alkanoylamino, nitro, trifluoromethyl and halogen.
and wherein the aryl and the aryl moiety of the arovl and the arylsulfonyl are selected from the group consisting of benzyl and naphthyl, and the heteroaryl and the heteroaryl moiety of the heteroarylcarbonyl and the heteroarylsulfonyl are selected from the group consisting of pyridyl, pyrazyl, pyrimidinyl, pyridazinyl, pyrrolyl, imidazolyl, pyraolyl, oxazolyl, thiazolyl, furyl, thienyl guinolyl, benzimidazolyl and indolyl,
or a pharmaceutically acceptable salt thereof.

**2.** A propenone derivative or a pharmaceutically acceptable salt thereof according to Claim 1, wherein $R^1$ and $R^2$ each represents hydrogen.

**3.** A propenone derivative or a pharmaceutically acceptable salt thereof according to Claim 2, wherein $R^3$ represents hydrogen or $C_1$-$C_6$ alkyl.

**4.** A propenone derivative or a pharmaceutically acceptable salt thereof according to Claim 3, wherein $R^4$, $R^5$, $R^6$ and $R^7$ represent independently hydrogen or $C_1$-$C_6$ alkyl.

**5.** (E)-3-(Indol-3-yl)-1-(3,4,5-trimethoxyphenyl)-2-propen-1-one, (E)-3-(indol-3-yl)-2-methyl-1-(3,4,5-trimethoxyphenyl) -2-propen-1-one, (E)-2-methyl-3-(6-methylindol-3-yl)-1-(3,4,5-trimethoxyphenyl)-2-propen-1-one, (E)-3-(6-ethylindol-3-yl)-2-methyl-1-(3,4,5-trimethoxyphenyl)-2-propen-1-one or pharmaceutically acceptable salts thereof.

**6.** A propenone derivative or a pharmaceutically acceptable salt thereof according to Claim 1, wherein $R^1$ represents substituted or unsubstituted aroyl.

**7.** (E)-3-[1-(3,4,5-Trimethoxybenzoyl)indol-3-yl]-1-(3,4,5-trimethoxyphenyl)-2-propen-1-one or a pharmaceutically acceptable salt thereof.

**8.** A pharmaceutically composition comprising a pharmaceutically acceptable carrier and an effective amount of a compound according to Claims 1 to 7 as an active ingredient.

**Patentansprüche**

**1.** Propenonderivat der nachstehenden Formel (I):

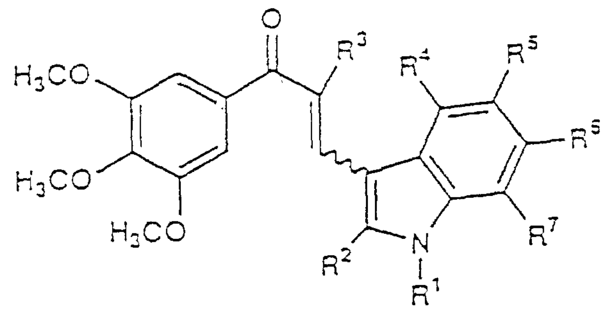

(I)

wobei $R^1$ ein Wasserstoffatom, einen $C_1$-$C_6$-Alkyl-, $C_1$-$C_7$-Alkanoyl-, $C_1$-$C_6$-Alkoxycarbonyl-, $C_1$-$C_6$-Alkylsulfonyl-, $C_7$-$C_{13}$-Aralkylrest, einen substituierten oder unsubstituierten Aroylrest, einen substituierten oder unsubstituierten Arylsulfonylrest, einen substituierten oder unsubstituierten Heteroarylcarbonylrest, einen substituierten oder unsubstituierten Heteroarylsulfonylrest, einen Diglycolylrest oder

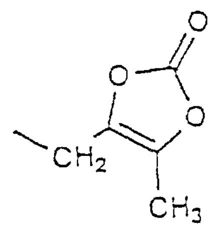

bedeutet; $R^2$ ein Wasserstoffatom, einen $C_1$-$C_6$-Alkylrest, ein Halogenatom, einen substituierten oder unsubstituierten Arylrest oder einen substituierten oder unsubstituierten Heteroarylrest bedeutet; $R^3$ ein Wasserstoffatom, einen $C_1$-$C_6$-Alkylrest oder einen substituierten oder unsubstituierten Arylrest bedeutet; und $R^4$, $R^5$, $R^6$ und R7 unabhängig voneinander ein Wasserstoffatom, einen $C_1$-$C_6$-Alkyl-, $C_1$-$C_6$-Alkoxy-, $C_7$-$C_{13}$-Aralkyloxyrest, eine Hydroxy-,Nitrogruppe, ein Halogenatom, eine Trifluormethylgruppe oder einen Rest der Formel -$NR^8R^9$ bedeuten (wobei $R^8$ und $R^9$ unabhängig voneinander ein Wasserstoffatom, einen $C_1$-$C_6$-Alkyl-, $C_1$-$C_7$-Alkanoyl-, $C_1$-$C_6$-Alkoxycarbonylrest oder einen substituierten oder unsubstituierten Aroylrest bedeuten) oder ein pharmazeutsich verträgliches Salz davon,
wobei die vorstehenden substituierten Reste 1 bis 3 Substituenten haben, die aus der Gruppe bestehend aus $C_1$-$C_6$-Alkyl-, $C_1$-$C_6$-Alkoxy-, $C_1$-$C_7$-Alkanoyl-, $C_1$-$C_6$-Alkoxycarbonylresten, einer Hydroxy-, Aminogruppe, $C_1$-$C_6$-Alkylamino-, Di($C_1$-$C_6$-alkyl)amino-, $C_1$-$C_7$-Alkanoylaminoresten, einer Nitro-, Trifluormethylgruppe und Halogenatomen ausgewählt sind,
und wobei der Arylrest und der Arylanteil des Aroyl- und des Arylsulfonylrestes aus einer Benzyl- und Naphthylgruppe ausgewählt ist, und der Heteroarylrest und der Heteroarylanteil des Heteroarylcarbonyl- und der Heteroarylsulfonylrestes aus einer Pyridyl-, Pyrazyl-, Pyrimidinyl-, Pyridazinyl-, Pyrrolyl-, Imidazolyl-, Pyrazolyl-, Oxazolyl-, Thiazolyl-, Furyl-, Thienyl-, Chinolyl-, Benzimidazolyl- und Indolylgruppe ausgewählt ist, oder ein pharmazeutisch verträgliches Salz davon.

**2.** Propenonderivat oder ein pharmazeutisch verträgliches Salz davon nach Anspruch 1, wobei $R^1$ und $R^2$ jeweils ein Wasserstoffatom bedeuten.

**3.** Propenonderivat oder ein pharmazeutisch verträgliches Salz davon nach Anspruch 2, wobei $R^3$ ein Wasserstoffatom oder einen $C_1$-$C_6$-Alkylrest bedeutet.

**4.** Propenonderivat oder ein pharmazeutisch verträgliches Salz davon nach Anspruch 3, wobei $R^4$, $R^5$, $R^6$ und $R^7$ unabhängig voneinander ein Wasserstoffatom oder einen $C_1$-$C_6$-Alkylrest bedeuten.

**5.** (E)-3-(Indol-3-yl)-1-(3,4,5-trimethoxyphenyl)-2-propen-1-on, (E)-3-(Indol-3-yl)-2-methyl-1-(3,4,5-trimethoxyphenyl)-2-propen-1-on, (E)-2-Methyl-3-(6-methylindol-3-yl)-1-(3,4,5-trimethoxyphenyl)-2-propen-1-on, (E)-3-(6-Ethylindol-3-yl)-2-methyl-1-(3,4,5-trimethoxyphenyl)-2-propen-1-on oder ein pharmazeutisch verträgliches Salz davon.

**6.** Propenonderivat oder ein pharmazeutische verträgliches Salz davon nach Anspruch 1, wobei $R^1$ einen substituierten oder unsubstituierten Aroylrest bedeutet.

**7.** (E)-3-[1-(3,4,5-Trimethoxybenzoyl)indol-3-yl]-1-(3,4,5-trimethoxyphenyl)-2-propen-1-on oder ein pharmazeutisch verträgliches Salz davon.

**8.** Arzneimittel umfassend einen pharmazeutisch verträglichen Träger und eine wirksame Menge einer Verbindung nach einem der Ansprüche 1 bis 7 als Wirkstoff.

## Revendications

**1.** Dérivé de type propénone, représenté par la formule (I) suivante :

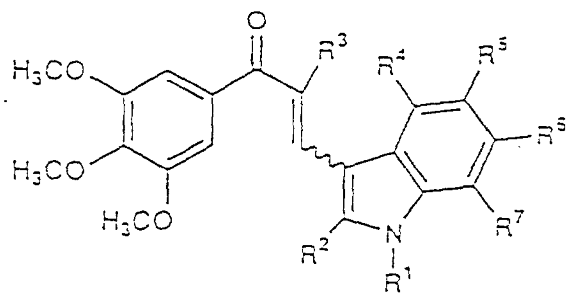

(I)

dans laquelle

$R^1$ représente un atome d'hydrogène, ou un groupe alkyle en $C_{1-6}$, alcanoyle en $C_{1-7}$, (alcoxy en $C_{1-6}$)carbonyle, alkylsulfonyle en $C_{1-6}$, aralkyle en $C_{7-13}$, aroyle substitué ou non, arylsulfonyle substitué ou non, hétéroarylcarbonyle substitué ou non, hétéroarylsulfonyle substitué ou non, diglycolyle ou

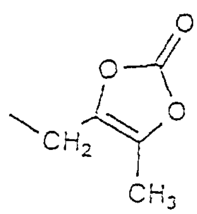

;

$R^2$ représente un atome d'hydrogène ou d'halogène, ou un groupe alkyle en $C_{1-6}$, aryle substitué ou non ou hétéroaryle substitué ou non ;
$R^3$ représente un atome d'hydrogène, ou un groupe alkyle en $C_{1-6}$ ou aryle substitué ou non ;
et $R^4$, $R^5$, $R^6$ et $R^7$ représentent chacun, indépendamment, un atome d'hydrogène ou d'halogène, un groupe alkyle en $C_{1-6}$, alcoxy en $C_{1-6}$, aralcoxy en $C_{7-13}$, hydroxy, nitro ou trifluorométhyle, un groupe de formule $-NR^8R^9$ où $R^8$ et $R^9$ représentent chacun, indépendamment, un atome d'hydrogène, ou un groupe alkyle en $C_{1-6}$, alcanoyle en $C_{1-7}$, (alcoxy en $C_{1-6}$)carbonyle ou aroyle substitué ou non ;
les groupes substitués mentionnés ci-dessus portant de 1 à 3 substituants choisis dans l'ensemble formé par les groupes alkyle en $C_{1-6}$, alcoxy en $C_{1-6}$, alcanoyle en $C_{1-7}$, (alcoxy en $C_{1-6}$)carbonyle, hydroxy, amino, (alkyle en $C_{1-6}$)amino, di(alkyle en $C_{1-6}$)amino, (alcanoyle en $C_{1-7}$)amino, nitro et trifluorométhyle et les atomes d'halogène,
et le groupe aryle et les fragments aryle des groupes aroyle et arylsulfonyle étant choisis dans l'ensemble formé par les groupes phényle et naphtyle, et le groupe hétéroaryle et les fragments hétéroaryle des groupes hétéroarylcarbonyle et hétéroarylsulfonyle étant choisis dans l'ensemble formé par les groupes pyridyle, pyrazinyle, pyrimidinyle, pyridazinyle, pyrrolyle, imidazolyle, pyrazolyle, oxazolyle, thiazolyle, furyle, thiényle, quinolyle, benzimidazolyle et indolyle ;

ou sel d'un tel dérivé, admissible en pharmacie.

2. Dérivé de type propénone ou sel admissible en pharmacie d'un tel dérivé, conforme à la revendication 1, dans lequel $R^1$ et $R^2$ représentent chacun un atome d'hydrogène.

3. Dérivé de type propénone ou sel admissible en pharmacie d'un tel dérivé, conforme à la revendication 2, dans lequel $R^3$ représente un atome d'hydrogène ou un groupe alkyle en $C_{1-6}$,

4. Dérivé de type propénone ou sel admissible en pharmacie d'un tel dérivé, conforme à la revendication 3, dans lequel $R^4$, $R^5$, $R^6$ et $R^7$ représentent chacun, indépendamment, un atome d'hydrogène ou un groupe alkyle en $C_{1-6}$.

5. (E)-3-(Indol-3-yl)-1-(3,4,5-triméthoxyphényl)-2-propène-1-one, (E)-3-(indol-3-yl)-2-méthyl-1-(3,4,5-triméthoxyphényl)-2-propène-1-one, (E)-2-méthyl-3-(6-méthyl-indol-3-yl)-1-(3,4,5-triméthoxyphényl)-2-propène-1-one, (E)-3-(6-éthyl-indol-3-yl)-2-méthyl-1-(3,4,5-triméthoxyphényl)-2-propène-1-one, et leurs sels admissibles en pharmacie.

6. Dérivé de type propénone ou sel admissible en pharmacie d'un tel dérivé, conforme à la revendication 1, dans lequel $R^1$ représente un groupe aroyle substitué ou non.

7. (E)-3-[1-(3,4,5-triméthoxybenzoyl)indol-3-yl)-1-(3,4,5-triméthoxyphényl)-2-propène-1-one, et ses sels admissibles en pharmacie.

8. Composition pharmaceutique comprenant un véhicule admissible en pharmacie et, comme ingrédient actif, un composé conforme à l'une des revendications 1 à 7, en une quantité suffisante pour qu'il soit efficace.